# EUROPEAN PATENT SPECIFICATION

(11) **EP 2 375 900 B1**
(45) Date of publication and mention of the grant of the patent: **02.03.2016**
(21) Application number: 09832613.5
(22) Date of filing: 11.12.2009
(51) Int. Cl.: A61K 31/495, A61P 25/00

(54) **METHODS FOR TREATING MULTIPLE SCLEROSIS USING TETRACYCLIC PYRAZINOINDOLES**
VERFAHREN ZUR BEHANDLUNG VON MULTIPLER SKLEROSE MIT TETRACYCLISCHEN PYRAZINOINDOLEN
MÉTHODES DE TRAITEMENT DE LA SCLÉROSE EN PLAQUES UTILISANT DES PYRAZINOINDOLES TÉTRACYCLIQUES

(30) Priority: 12.08.2009 US 233235 P; 11.12.2008 US 121574 P
(43) Date of publication of application: 19.10.2011
(73) Proprietor: Biovista, Inc., Charlottesville, VA 22903 (US)
(72) Inventor: DEFTEREOS, Spyros, GR-15342 Athens (GR); PERSIDIS, Andreas, GR-16673 Athens (GR)
(74) Representative: Elsy, David
(86) International application number: PCT/US2009/067673
(87) International publication number: WO 2010/068867

(56) References cited:
- WO-A2-2006/048242
- US-A- 3 959 470
- US-A- 5 538 980
- US-A1- 2007 027 178
- US-A1- 2007 270 429
- US-B2- 7 199 151
- GONSETTE ET AL: "Neurodegeneration in multiple sclerosis: The role of oxidative stress and excitotoxicity", JOURNAL OF NEUROLOGICAL SCIENCES, ELSEVIER SCIENTIFIC PUBLISHING CO, AMSTERDAM, NL, vol. 274, no. 1-2, 15 November 2008 (2008-11-15), pages 48-53, XP025587925, ISSN: 0022-510X, DOI: 10.1016/J.JNS.2008.06.029 [retrieved on 2008-08-05]
- BOLAND A ET AL: "Pirlindole and dehydropirlindole protect rat cultured neuronal cells against oxidative stress-induced cell death through a mechanism unrelated to MAO-A inhibition", BRITISH JOURNAL OF PHARMACOLOGY, vol. 135, no. 3, February 2002 (2002-02), pages 713-720, XP002671355, ISSN: 0007-1188
- FARRELL R ET AL: "EMERGING THERAPIES IN MULTIPLE SCLEROSIS", EXPERT OPINION ON EMERGING DRUGS, ASHLEY PUBLICATIONS, GB, vol. 10, no. 4, 1 January 2005 (2005-01-01), pages 797-816, XP008067911, ISSN: 1472-8214
- Anonymous: "Biovista announces positive efficacy results in a pre-clinical trial of BVA-201 for Multiple Sclerosis", , 9 September 2009 (2009-09-09), XP002671356, Retrieved from the Internet: URL:http://www.biovista.com/print_article. php?pid=136&parent=http://www.biovista.com /news.php?article_id=136&year=2009 [retrieved on 2012-03-09]
- Anonymous: "Biovista Inc. announces positive efficacy results in a pre-clinical trial of BVA-101 for Multiple Sclerosis", , 2 April 2009 (2009-04-02), XP002671357, Retrieved from the Internet: URL:http://www.biovista.com/print_article. php?pid=131&parent=http://www.biovista.com /news.php?article_id=131&year=2009 [retrieved on 2012-03-09]

### SEQUENCE LISTING

<110> BIOVISTA, INC.
   DEFTEREOS, Spyros
   PERSIDIS, Andreas
<120> METHODS FOR TREATING MULTIPLE SCLEROSIS USING TETRACYCLIC PYRAZINOINDOLES
<130> 46892-216941
<150> 61/121,574
<151> 2008-12-11
<150> 61/233,235
<151> 2009-08-12
<160> 2
<170> PatentIn version 3.5
<210> 1
<211> 21
<212> PRT
<213> Mouse
<400> 1
   Met Glu Val Gly Trp Tyr Arg Ser Pro Phe Ser Arg Val Val His Leu
   1 5 10 15
   Tyr Arg Asn Gly Lys
   20
<210> 2
<211> 21
<212> PRT
<213> Homo sapiens
<400> 2

### SEQUENCE LISTING

<110> BIOVISTA, INC.
   DEFTEREOS, Spyros
   PERSIDIS, Andreas
<120> METHODS FOR TREATING MULTIPLE SCLEROSIS USING TETRACYCLIC PYRAZINOINDOLES
<130> 46892-216941
<150> 61/121,574
<151> 2008-12-11
<150> 61/233,235
<151> 2009-08-12
<160> 2
<170> PatentIn version 3.5
<210> 1
<211> 21
<212> PRT
<213> Mouse
<400> 1
   Met Glu Val Gly Trp Tyr Arg Ser Pro Phe Ser Arg Val Val His Leu
   1 5 10 15
   Tyr Arg Asn Gly Lys
   20
<210> 2
<211> 21
<212> PRT
<213> Homo sapiens
<400> 2

## Description

### TECHNICAL FIELD

The invention described herein relates to compounds for use in treating multiple sclerosis. In particular, the invention relates to compounds for use in treating multiple sclerosis comprising one or more tetracyclic pyrazinoindoles, and/or pharmaceutically acceptable salts thereof.

### BACKGROUND AND SUMMARY OF THE INVENTION

Multiple sclerosis (MS) is an inflammatory demyelinating disease of the central nervous system (CNS). MS may take several forms, with new symptoms occurring either in discrete attacks (relapsing forms) or slowly accumulating over time (progressive forms). Between attacks, though symptoms may disappear completely, often permanent neurological problems yet occur, especially as the disease advances. Even so, symptoms of MS usually appear in episodic acute periods that may worsen upon recurrence (relapses, exacerbations, bouts, and/or attacks). With such recurrence, the condition may develop into a gradually-progressive deterioration of neurologic function. Many subtypes of MS present in a combination of both acute episodes and gradual deterioration. One common presentation of MS is the clinically isolated syndrome (CIS), in which a patient has an attack suggestive of demyelination, but does not fulfill the criteria for multiple sclerosis. It has been reported that only 30 to 70% of persons experiencing CIS later develop MS.

When MS does develop, several specific subtypes, or patterns of progression of the disease, have been described. The United States National Multiple Sclerosis Society has standardized four subtype definitions as relapsing remitting (RR), secondary progressive (SP), primary progressive (PP), and progressive relapsing (PR). Those subtypes are characterized by using the past course of the disease as one means of predicting the future course. It is appreciated that the accurate characterization of the subtype may be useful in both the prognosis of the disease and also in therapeutic decisions.

The relapsing-remitting MS subtype is characterized by unpredictable relapses which may be followed by periods of relative quiet (remission) with no new signs of disease activity. Such periods of relative quiet may last months or even years. The deficits suffered during attacks may either resolve or leave sequelae. The relapsing-remitting subtype is the most common subtype and describes the initial course of 85-90% of individuals with MS. When deficits always resolve between attacks, this subtype may also be referred to as benign MS.

The secondary progressive MS subtype also includes initial relapsing-remitting MS, but where the afflicted patient then begins to have progressive neurological decline between acute attacks without any definite periods of remission. However, occasional relapses and minor remissions may appear. The median time between disease onset and conversion from relapsing-remitting to secondary progressive MS has been reported to be 19 years.

The primary progressive MS subtype describes the approximately 10-15% of individuals who never have remission after their initial MS symptoms. The subtype is further characterized by progression of disability from onset, with no, or only occasional and minor, remissions and improvements. Generally, the age of onset for the primary progressive subtype is later than other subtypes.

The progressive relapsing MS subtype is characterized by those individuals who, from onset, have a steady neurological decline but also suffer clear superimposed attacks. This subtype is the least common of all subtypes.

In addition to the standard subtypes, cases with non-standard behavior have also been described. Those non-standard subtypes are sometimes referred to as borderline forms of multiple sclerosis, and include Devic's disease, Balo concentric sclerosis, Schilder's diffuse sclerosis, and Marburg multiple sclerosis.

MS is a major cause of disability, because in most patients the disease ultimately has a progressive course. In most patients, the progressive course of the disease manifests itself during or after a preceding phase of relapses and remissions (secondary progressive disease), whereas in a small percentage of patients (10-15%) the disease course is progressive from onset (primary progressive disease). Most currently available treatments for multiple sclerosis are aimed at suppressing the inflammatory component of the disease. Their main clinical impact is on relapses whereas an effect on permanent disability is less well established. Patients with primary progressive MS show less inflammatory activity, which is one of the reasons why they are frequently excluded from treatment trials, despite clear clinical progression. Recent evidence suggests that axonal loss may occur earlier in the disease course of MS than previously anticipated. Further, such early axonal loss may be the pathologic correlate of

irreversible disability. MS is also frequently characterized by plaques or lesions of demyelination in the nerve fibers of the brain and spinal cord. Demyelination causes multiple and varied neurological symptoms and signs, usually with relapses and exacerbations.

The clinical course of MS is highly variable and unpredictable, with many patients experiencing acute episodes of exacerbations, followed by periods of remission. The disease progresses at various paces to a chronic, degenerative condition. Frequently, a diagnosis of MS may not be made for many years after the onset of symptoms because the symptoms can be variable, sporadic, and similar to those associated with other disorders. As the disease progresses, patients are frequently unable to remain fully ambulatory, and their functional systems steadily decline. The most severe cases of MS are characterized by paralysis or even death.

The precise causes of MS are not yet known, though several theories have been proposed. Research to date has indicated that the etiology of MS may in fact be related to a combination of factors, such as autoimmunity, environmental, viral and genetic factors. Thus, there remains a need to identify additional treatments for MS which can treat the disease, minimize the effects of the disease, and/or slow the progression of the disease.

Gonsette RE (J. Neur.Sci (2008) 274, 48-53) discusses the role of oxiditive stress in mediating neurodegeration in multiple sclerosis.

Boland et al (J.J.P. (2002) 135, 713-720) discusses pirlindole and dehydropirlindole in the protection of neuronal cells agains oxiditive stress-induced cell death.

It has been discovered that tetracyclic pyrazinoindoles, and pharmaceutically acceptable salts thereof, are useful in treating patients suffering from or in need of relief from multiple sclerosis. The use of tetracyclic pyrazinoindoles, or pharmaceutically acceptable salts thereof, in treating multiple sclerosis has heretofore been unknown.

There is described a tetracyclic pyrazinoindole or a pharmaceutically acceptable salt thereof for use in treating multiple sclerosis.

The use described includes the use of a therapeutically effective amount of one or more tetracyclic pyrazinoindoles and/or pharmaceutically acceptable salts thereof, and using one or more dimebolins and/or pharmaceutically acceptable salts thereof.

The one or more tetracyclic pyrazinoindoles and/or pharmaceutically acceptable salts thereof, may be co-administered with one or more NMDA receptor antagonists.

The one or more tetracyclic pyrazinoindoles and/or pharmaceutically acceptable salts thereof, may be co-administered with one or more HMG-CoA reductase inhibitors, also referred to as statins, to a patient suffering from, or in need of relief from one or more forms of multiple sclerosis, or borderline forms of multiple sclerosis.

The one or more tetracyclic pyrazinoindoles and/or pharmaceutically acceptable salts thereof, may be co-administered with one or more antipsychotic compounds and/or atypical antipsychotic compounds.

The one or more tetracyclic pyrazinoindoles and/or pharmaceutically acceptable salts thereof, may be co-administered with one or more immunosuppressive drugs and/or one or more immunomodulatory drugs.

It is to be understood that in each of the foregoing methods, all combinations of compounds described herein may be co-administered, such as but not limited to administering a therapeutically effective amount of one or more tetracyclic pyrazinoindoles and/or pharmaceutically acceptable salts thereof, and administering a therapeutically effective amount of one or more dimebolins and/or pharmaceutically acceptable salts thereof, and administering a therapeutically effective amount of one or more NMDA receptor antagonists to a patient.

There is provided, in a first aspect of the invention, a compound according to the formula 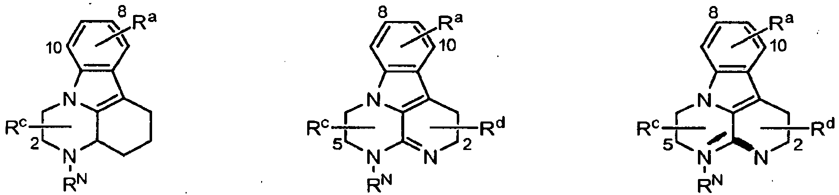 or a pharmaceutically acceptable salt thereof; for use in the treatment of multiple sclerosis wherein
R^{a} is hydrogen, or R^{a} represents 1 to 4 substituents, each independently selected from halo and hydroxy, and alkoxy, acyloxy, alkyl, heteroalkyl, cycloalkyl, cycloheteroalkyl, aryl, heteroaryl, arylalkyl, or heteroarylalkyl, each of which is optionally substituted;
R^{c} is hydrogen, or R^{c} represents 1 or 2 substituents, each independently selected from alkyl, heteroalkyl, cycloalkyl, cycloheteroalkyl, aryl, heteroaryl, arylalkyl, and heteroarylalkyl, each of which is optionally substituted;
R^{d} is hydrogen, or R^{c} represents 1 or 2 substituents, each independently selected from alkyl, heteroalkyl, cycloalkyl, cycloheteroalkyl, aryl, heteroaryl, arylalkyl, and heteroarylalkyl, each of which is optionally substituted; and
R^{N} is hydrogen or hydroxy, or alkoxy, acyloxy, alkyl, heteroalkyl, cycloalkyl, cycloheteroalkyl, aryl, heteroaryl, arylalkyl, or heteroarylalkyl, each of which is optionally substituted; or R^{N} and the attached nitrogen form an amide, carbamate, or urea, or thiono derivative thereof;

In one embodiment, the compositions are useful for treating any form, or combination of forms of MS, including primary progressive MS, secondary progressive MS, relapsing remitting MS, and/or progressive relapsing MS.- In another embodiment, the compositions described herein are useful for treating either progressive form of MS, including primary progressive MS and/or secondary progressive MS. In one aspect, the compositions described herein include a therapeutically effective amount of one or more compounds according to the first aspect of the invention and/or pharmaceutically acceptable salts thereof to a patient suffering from, or in need of relief from one or more forms of multiple sclerosis, or borderline forms of multiple sclerosis.

Also described herein not of the invention are compositions which include a therapeutically effective amount of one or more tetracyclic pyrazinoindoles and/or pharmaceutically acceptable salts thereof. In another embodiment, the compositions include a therapeutically effective amount of one or more tetracyclic pyrazinoindoles and/or pharmaceutically acceptable salts thereof, and a therapeutically effective amount of one or more dimebolins and/or pharmaceutically acceptable salts thereof. In an embodiment of the invention, the compositions include a therapeutically effective amount of one or more compounds according to the first aspect of the invention and/or pharmaceutically acceptable salts thereof, and a therapeutically effective amount of one or more NMDA receptor antagonists. In another embodiment, the compositions include a therapeutically effective amount of one or more compounds according to the first aspect of the invention and/or pharmaceutically acceptable salts thereof, and a therapeutically effective amount of one or more HMG-CoA reductase inhibitors, also referred to as statins. In another embodiment, the compositions include a therapeutically effective amount of one or more compounds according to the first aspect of the invention and/or pharmaceutically acceptable salts thereof, and a therapeutically effective amount of one or more immunosuppressive drugs and/or one or more immunomodulatory drugs. It is to be understood that in each of the foregoing compositions, all combinations of compounds described herein may be included in the compositions, such as but not limited to a therapeutically effective amount of one or more compounds according to the first aspect of the invention and/or pharmaceutically acceptable salts thereof, and a therapeutically effective amount of one or more dimebolins and/or pharmaceutically acceptable salts thereof, and a therapeutically effective amount of one or more NMDA receptor antagonists to a patient.

Also described are kits or packages. Kits and packages include preparations where the co-administered compounds are placed in a format following the dosing protocols described herein. For example, a package may include a grid pattern, wherein each section includes a dual or triple bubble pack for the one or more tetracyclic pyrazinoindole dosages, and the dimebolin dosage, NMDA antagonist dosage, statin dosage, the immunosuppressive drug dosage, and/or the immunomodulatory drug dosage. It is appreciated that other configurations that include other combinations of one or more of the dimebolin dosage, NMDA antagonist dosage, statin dosage, the immunosuppressive drugs, the immunomodulatory drugs are described herein.

### BRIEF DESCRIPTION OF THE DRAWINGS

FIG. 1 Treatment of EAE induction animal model. (A) vehicle-Group; (B) dexamethasone-Group; (C) BVA-201, 1 mg/kg-Group; (F) BVA-201, 30 mg/kg-Group.
FIG. 2 Weight loss during treatment of EAE induction animal model as a measure of gross toxicity. (A) vehicle-Group; (B) dexamethasone-Group; (C) BVA-201, 1 mg/kg-Group; (F) BVA-201, 30 mg/kg-Group.
FIG. 3. Histology mean group severity score. (A) vehicle-Group; (B) dexamethasone-Group; (C) BVA-201, 1 mg/kg-Group; (F) BVA-201, 30 mg/kg-Group.
FIG. 4. Histology correlation between % of WMD and clinical score at termination.
FIG. 5. Histology mean % of white matter damage. (A) vehicle-Group; (B) dexamethasone-Group; (C) BVA-201, 1 mg/kg-Group; (F) BVA-201, 30 mg/kg-Group.

### DETAILED DESCRIPTION

Described herein are new methods for treating MS by administering a therapeutically effective amount of one or more tetracyclic pyrazinoindoles, or pharmaceutically acceptable salts thereof. Also described herein are compositions for treating MS, where the compositions include a therapeutically effective amount of one or more tetracyclic pyrazinoindoles, or pharmaceutically acceptable salts thereof.

In one embodiment, pharmaceutical compositions are described herein. Illustrative pharmaceutical compositions include dosage forms of one or more compounds according to the first aspect of the invention and/or pharmaceutically acceptable salts thereof, and one or more pharmaceutically acceptable carriers, excipients, and/or diluents therefor. Other illustrative pharmaceutical compositions include (a) mixtures of one or more compounds according to the first aspect of the invention and/or pharmaceutically acceptable salts thereof, and one or more dimebolins and/or pharmaceutically acceptable salts thereof, (b) mixtures of one or more compounds according to the first aspect of the invention and/or pharmaceutically acceptable salts thereof, and one or more NMDA antagonists, (c) mixtures of one or more compounds according to the first aspect of the invention and/or pharmaceutically acceptable salts thereof, and one or more statins, (d) mixtures of one or more compounds according to the first aspect of the invention and/or pharmaceutically acceptable salts thereof, and one or more immunosuppressive drugs, (e) mixtures of one or more compounds according to the first aspect of the invention and/or pharmaceutically acceptable salts thereof, and one or more immunomodulatory drugs, (f) mixtures of one or more compounds according to the first aspect of the invention and/or pharmaceutically acceptable salts thereof, one or more dimebolins and/or pharmaceutically acceptable salts thereof, and one or more NMDA antagonists. Other illustrative formulations include "sandwich" formulations where two or more separate drug dosage forms are conveniently adhered one to the other for simultaneous co-administration.

In another illustrative embodiment, the methods include the step of administering a therapeutically effective amount of one or more compounds according to the first aspect of the invention, or pharmaceutically acceptable salts thereof; and/or administering a pharmaceutical composition, such as the compositions described herein, comprising a therapeutically effective amount of one or more compounds according to the first aspect of the invention, or pharmaceutically acceptable salts thereof to a patient suffering from or in need of relief from MS.

In addition, described herein are tetracyclic pyrazinoindoles of formulae (I), (Ia), (Ib), (Ic), (II), (III), and (IV). The formulae include various functional groups on aromatic rings, such as R^{a}. It is to be understood that derivatives of those compounds also include the compounds having for example different functional groups on those aromatic rings than those explicitly set forth in the definition of formulae (I), (Ia), (Ib), (Ic), (II), (III), and (IV). In addition, it is to be understood that derivatives of those compounds also include the compounds having those same or different functional groups at different positions on the aromatic ring. Similarly, derivatives include parallel variations of other functional groups on the compounds described herein, such as R^{N}. In addition, as used herein the term tetracyclic pyrazinoindole also refers to the corresponding prodrug derivatives of the compounds described herein, and including prodrugs of the various analogs and derivatives thereof.

It is also to be understood that analogs of the tetracyclic pyrazinoindoles described herein, which are structurally similar, or are similar based on biochemical and/or biological activity, whether or not they are also tetracyclic pyrazinoindoles, are also included in the term. Analogs include, but are not limited to, the corresponding ring expanded or ring contracted core structures of the compounds described herein, such as but not limited to the corresponding cycloheptane, cyclopentane, homopiperidine and pyrrolidine. Other analogs include, but are not limited to, the corresponding ring systems that include additional heteroatoms, such as the corresponding pyrimidine, azaindole and pyridazine. Therefore, it is to be understood that all such analogs compounds are also considered to be tetracyclic pyrazinoindoles.

In one embodiment the invention there is provided a compound of formula (I) 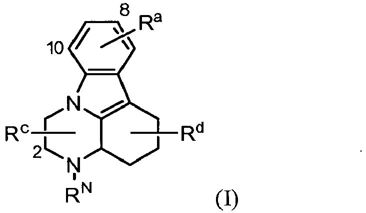 or a pharmaceutically acceptable salt thereof, wherein
R^{a} is hydrogen, or R^{a} represents 1 to 4 substituents, each independently selected from halo and hydroxy, and alkoxy, acyloxy, alkyl, heteroalkyl, cycloalkyl, cycloheteroalkyl, aryl, heteroaryl, arylalkyl, or heteroarylalkyl, each of which is optionally substituted;
R^{c} is hydrogen, or R^{c} represents 1 or 2 substituents, each independently selected from alkyl, heteroalkyl, cycloalkyl, cycloheteroalkyl, aryl, heteroaryl, arylalkyl, and heteroarylalkyl, each of which is optionally substituted;
R^{d} is hydrogen, or R^{d} represents 1 or 2 substituents, each independently selected from alkyl, heteroalkyl, cycloalkyl, cycloheteroalkyl, aryl, heteroaryl, arylalkyl, and heteroarylalkyl, each of which is optionally substituted; and
R^{N} is hydrogen or hydroxy, or alkoxy, acyloxy, alkyl, heteroalkyl, cycloalkyl, cycloheteroalkyl, aryl, heteroaryl, arylalkyl, or heteroarylalkyl, each of which is optionally substituted; or R^{N} and the attached nitrogen form an amide, carbamate, or urea, or thiono derivative thereof.

In another embodiment, R^{a} is hydrogen. In another embodiment, R^{a} represents 1 to 4 substituents, each independently selected from halo (including F, Cl, and Br), optionally substituted hydroxy (including alkoxy and acyloxy), acyl (including C(O)NHNH₂, CO₂Et), alkyl (including methyl), heteroalkyl, cycloalkyl (including cyclohexyl, cyclododecyl, adamantyl, and the like), aryl (including 4-methoxyphenyl), and arylalkyl, each of which is optionally substituted. In another embodiment, R^{c} is hydrogen. In another embodiment, R^{c} represents 1 or 2 substituents, each independently selected from alkyl and aryl (including phenyl), each of which is optionally substituted. In another embodiment, R^{N} is H or acyl. In another embodiment, R^{N} is alkyl, heteroalkyl, aryl, or arylalkyl, each of which is optionally substituted.

In another embodiment, there is provided a compound of formula (Ia) 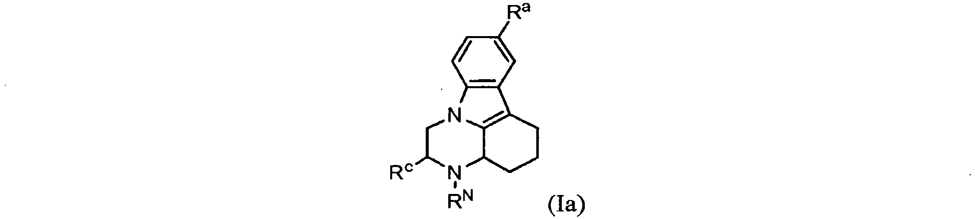 or a pharmaceutically acceptable salt thereof, wherein
R^{a} is hydrogen, halo, or hydroxy, or alkoxy, acyloxy, alkyl, heteroalkyl, cycloalkyl, cycloheteroalkyl, aryl, heteroaryl, arylalkyl, or heteroarylalkyl, each of which is optionally substituted;
R^{c} is hydrogen, or alkyl, heteroalkyl, cycloalkyl, cycloheteroalkyl, aryl, heteroaryl, arylalkyl, or heteroarylalkyl, each of which is optionally substituted; and
R^{N} is hydrogen or hydroxy, or alkoxy, acyloxy, alkyl, heteroalkyl, cycloalkyl, cycloheteroalkyl, aryl, heteroaryl, arylalkyl, or heteroarylalkyl, each of which is optionally substituted; or R^{N} and the attached nitrogen form an amide, carbamate, or urea, or thiono derivative thereof.

In another embodiment, R^{a} is selected from halo (including F, Cl, and Br), optionally substituted hydroxy (including methoxy, acetoxy), acyl (including C(O)NHNH₂, CO₂Et), alkyl (including methyl), heteroalkyl, cycloalkyl (including cyclohexyl, cyclododecyl, adamantyl, and the like), aryl (including 4-methoxyphenyl), or arylalkyl, each of which is optionally substituted. In another embodiment, R^{c} is selected from alkyl and aryl (including phenyl), each of which is optionally substituted. In another embodiment, R^{N} is H or acyl. In another embodiment, R^{N} is alkyl, heteroalkyl, aryl, or arylalkyl, each of which is optionally substituted.

In another embodiment, there is provided a compound of formula (Ib) 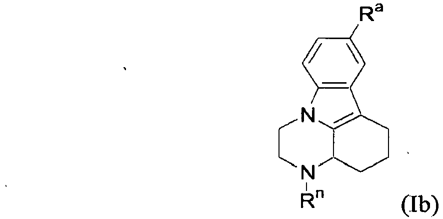 or a pharmaceutically acceptable salt thereof, wherein
R^{a} is hydrogen, halo, or hydroxy, or alkoxy, acyloxy, alkyl, heteroalkyl, cycloalkyl, cycloheteroalkyl, aryl, heteroaryl, arylalkyl, or heteroarylalkyl, each of which is optionally substituted; and
R^{N} is hydrogen or hydroxy, or alkoxy, acyloxy, alkyl, heteroalkyl, cycloalkyl, cycloheteroalkyl, aryl, heteroaryl, arylalkyl, or heteroarylalkyl, each of which is optionally substituted; or R^{N} and the attached nitrogen form an amide, carbamate, or urea, or thiono derivative thereof.

In another embodiment, R^{a} is selected from halo (including F, Cl, and Br), optionally substituted hydroxy (including methoxy, acetoxy), acyl (including C(O)NHNH₂, CO₂Et), alkyl (including methyl), heteroalkyl, cycloalkyl (including cyclohexyl, cyclododecyl, adamantyl, and the like), aryl (including 4-methoxyphenyl), or arylalkyl, each of which is optionally substituted. In another embodiment, R^{N} is H or acyl. In another embodiment, R^{N} is alkyl, heteroalkyl, aryl, or arylalkyl, each of which is optionally substituted.

In another embodiment, there is provided a compound of formula (Ib) 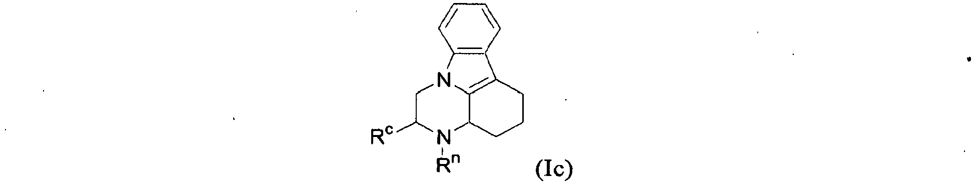 or a pharmaceutically acceptable salt thereof, wherein
R^{c} is hydrogen, or alkyl, heteroalkyl, cycloalkyl, cycloheteroalkyl, aryl, heteroaryl, arylalkyl, or heteroarylalkyl, each of which is optionally substituted; and
R^{N} is hydrogen or hydroxy, or alkoxy, acyloxy, alkyl, heteroalkyl, cycloalkyl, cycloheteroalkyl, aryl, heteroaryl, arylalkyl, or heteroarylalkyl, each of which is optionally substituted; or R^{N} and the attached nitrogen form an amide, carbamate, or urea, or thiono derivative thereof.

In another embodiment, R^{c} is selected from alkyl and aryl (including phenyl), each of which is optionally substituted. In another embodiment, R^{N} is H or acyl. In another embodiment, R^{N} is alkyl, heteroalkyl, aryl, or arylalkyl, each of which is optionally substituted.

In another embodiment of any of formulae (I) to (Ic), R^{N} is H. In another embodiment of any of formulae (I) to (Ic), R^{N} is alkyl, such as methyl, ethyl, butyl, and the like. In another embodiment of any of formulae (I) to (Ic), R^{N} is substituted alkyl, such as cyanomethyl, haloalkyl, hydroxyalkyl, aminoalkyl (including 2-aminoethyl, 2-diethylaminoethyl), hydroxyalkylaminoalkyl, aminoalkylhydroxyalkyl (including 4-methylpiperazinyl-2-hydroxypropyl, aminoalkylaminoalkyl, aminoalkylaminoalkylhydroxyalkyl (including Er₂N-(CH₂)₃-NH-CH₂-CH(OH)-CH₂, Me₂N-(CH₂)₄-NH-CH₂-CH(OH)-CH₂), aminocarbonylalkyl (including methylcarbonylmethyl, 2-aminocarbonylethyl, 3-methylpipidinylcarbonylmethyl), and the like. In another embodiment of any of formulae (I) to (Ic), R^{N} is aryl, such as 4-(3,4-dimethoxyphenyl)-2-thiazolyl and 1-methyl-3-nitro-1H-1,2,4-triazol-5-yl. In another embodiment of any of formulae (I) to (Ic), R^{N} is acyl, such as optionally substituted alkanoyl (including 7,7,7-trifluoroheptanoyl, diethylaminoacetyl), or optionally substituted benzoyl (including 3,4,5-trimethoxybenzoyl, 2-fluorobenzoyl).

In another embodiment of any of formulae (I) to (Ic), R^{a} is at C-8 (para to the nitrogen). In another embodiment of any of formulae (I) to (Ic), R^{a} is at C-8 and C-10 (including 8,10-dimethyl). In another embodiment of any of formulae (I) to (Ic), R^{a} represents 0 to 4 substituents selected from silyl (including trimethylsilyl, diphenylmethylsily, and the like), halo (including fluoro, chloro, and bromo), nitro, cyano, hydroxyl, alkyl (including methyl, ethyl, isopropyl and tertbutyl), cycloalkyl (including cyclopentyl, cycloheptyl, cyclodecanyl and adamantyl), cycloalkenyl (including cyclopentenyl, cyclohexenyl and cyclohexadienyl), hydroxyalkyl and hydroxycycloalkyl (including hydroxymethyl, phenylhydroxymethyl, hydroxycyclopentyl and dihydroxycyclohexyl), aryl (including phenyl and methoxyphenyl), alkoxy, alkoxyalkyl, cycloalkoxyl, and aryloxy (including methoxy, isobutoxy, cyclohexyloxy, benzyloxy, methoxyladamantyl, phenoxy, methoxylmethyl and methoxyphenoxy), amino (including NH₂, piperidinyl and quinuclidinyl), carbonyl, and carboxyl and derivatives thereof (including carboxy, acetoxy, ethoxycarbonyl and hydrazidocarbonyl), and others.

In another embodiment of any of formulae (I) to (Ic), R^{a} is at C-7. In another embodiment of any of formulae (I) to (Ic), R^{a} is at C-8. In another embodiment of any of formulae (I) to (Ic), R^{a} is at C-9. In another embodiment of any of formulae (I) to (Ic), R^{a} is at C-10. In another embodiment of any of formulae (I) to (Ic), R^{a} is at C-7 and C-10. In another embodiment of any of formulae (I) to (Ic), R^{a} is at C-8 and C-10. In another embodiment of any of formulae (I) to (Ic), R^{a} is selected from 9-chloro, 7-amino-10-methyl, 10-methyl, and 8,10-dimethyl. In another embodiment of any of formulae (I) to (Ic), R^{a} is methyl; and R^{c} is phenyl or 4-MeO-phenyl.

In another embodiment of any of formulae (I) to (Ic), R^{a} is one or more of the following:

| | | |
|---|---|---|
| trimethylsilyl | 1-adamantyl | cyclohexyloxy |
| fluoro | cyclopent-1-ene-yl | benzyloxy |
| chloro | cyclohex-1-ene-yl | phenoxy |
| bromo | cyclohexa-1,3-diene-yl | 4-MeO-phenoxy |
| nitro | hydroxymethyl | amino |
| hydroxy | phenyl-hydroxymethyl | (4-piperidinyl) |
| ethyl | 1-OH-1-cyclopentyl | 3-quinulidinyl |
| isopropyl | dihydroxycyclohexyl | hydrazidocarbonyl |
| tert-butyl | methoxymethyl | carboxy |
| 1-cyclopentyl | 2-MeO-2-adamantyl | acetoxy |
| cycloheptyl | methoxy | ethoxy carbonyl |
| cyclododecanyl | iso-butoxy | |

In another embodiment of any of formulae (I) to (Ic), R^{c} is at C-2. In another embodiment of any of formulae (I) to (Ic), R^{c} represents 0 to 2 substituents selected from alkyl (including methyl, ethyl and isopropyl) and aryl (including phenyl and methoxyphenyl). In another embodiment of any of formulae (I) to (Ic), R^{c} is at C-2.

In another embodiment, the compound is the corresponding dehydro compound of any of formulae (I) to (Ic), or embodiments thereof described herein.

In another embodiment, a therapeutically effective amount of pirlindole (also known as Pirazidol and/or pirlindolum, or 2,3,3a,4,5,6-hexahydro-8-methyl-1H-pyrazino[3,2,1-j,k]carbazole or 1,10-trimethylene-8-methyl-1,2,3,4-tetrahydropyrazino[1,2-a]indole) is included on the compositions described herein, or administered in the methods described herein. As used herein, pirlindole includes compounds of the formula: 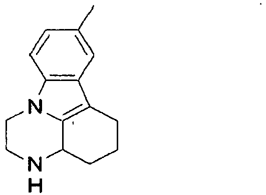 and pharmaceutically acceptable salts thereof, such as the hydrochloride salt or the mesylate salt. Pirlindole has been reported to be an antidepressant which acts as a reversible inhibitor of monoamine oxidase A (RIMA).

In another embodiment, there is provided a tetrindole including tetrindole (2,3,3a,4,5,6-Hexahydro-8-cyclohexyl-1H-pyrazino[3,2,1-j,k]carbazole) of the formula: 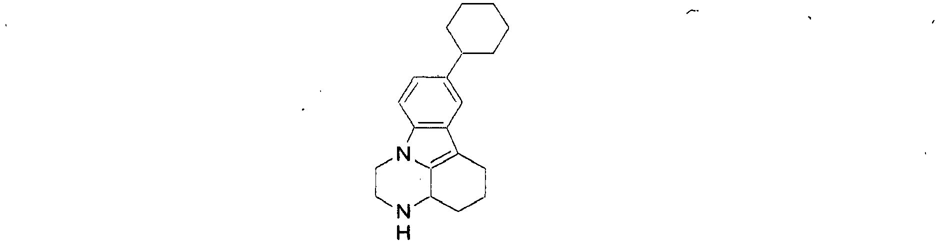 and pharmaceutically acceptable salts thereof, such as the mesylate salt.

In another embodiment, there is provided a compound of formulae (II), (III), or (IV) 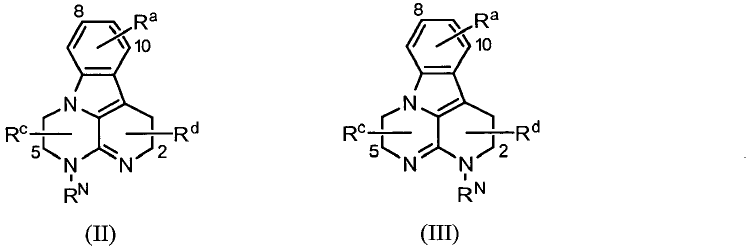 or a pharmaceutically acceptable salt thereof, wherein
R^{a} is hydrogen, or R^{a} represents 1 to 4 substituents, each independently selected from halo and hydroxy, and alkoxy, acyloxy, alkyl, heteroalkyl, cycloalkyl, cycloheteroalkyl, aryl, heteroaryl, arylalkyl, or heteroarylalkyl, each of which is optionally substituted;
R^{c} is hydrogen, or R^{c} represents 1 or 2 substituents, each independently selected from alkyl, heteroalkyl, cycloalkyl, cycloheteroalkyl, aryl, heteroaryl, arylalkyl, and heteroarylalkyl, each of which is optionally substituted;
R^{d} is hydrogen, or R^{d} represents 1 or 2 substituents, each independently selected from alkyl, heteroalkyl, cycloalkyl, cycloheteroalkyl, aryl, heteroaryl, arylalkyl, and heteroarylalkyl, each of which is optionally substituted; and
R^{N} is hydrogen or hydroxy, or alkoxy, acyloxy, alkyl, heteroalkyl, cycloalkyl, cycloheteroalkyl, aryl, heteroaryl, arylalkyl, and heteroarylalkyl, each of which is optionally substituted; or one or more R^{N} and the attached nitrogen form an amide, carbamate, or urea, or thiono derivative thereof.

In addition, described herein is a compound of formula (IV) 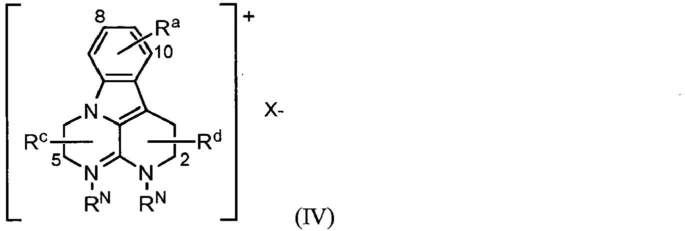 or a pharmaceutically acceptable salt thereof, wherein
R^{a} is hydrogen, or R^{a} represents 1 to 4 substituents, each independently selected from halo and hydroxy, and alkoxy, acyloxy, alkyl, heteroalkyl, cycloalkyl, cycloheteroalkyl, aryl, heteroaryl, arylalkyl, or heteroarylalkyl, each of which is optionally substituted;
R^{c} is hydrogen, or R^{c} represents 1 or 2 substituents, each independently selected from alkyl, heteroalkyl, cycloalkyl, cycloheteroalkyl, aryl, heteroaryl, arylalkyl, and heteroarylalkyl, each of which is optionally substituted;
R^{d} is hydrogen, or R^{d} represents 1 or 2 substituents, each independently selected from alkyl, heteroalkyl, cycloalkyl, cycloheteroalkyl, aryl, heteroaryl, arylalkyl, and heteroarylalkyl, each of which is optionally substituted;
R^{N} is independently selected in each instance from hydrogen and hydroxy, and alkoxy, acyloxy, alkyl, heteroalkyl, cycloalkyl, cycloheteroalkyl, aryl, heteroaryl, arylalkyl, and heteroarylalkyl, each of which is optionally substituted; or one or more R^{N} and the attached nitrogen form an amide, carbamate, or urea, or thiono derivative thereof; and
X is a pharmaceutically acceptable anion, such as chloride, mesylate, and the like.

In another embodiment, compounds of formulae (II), (III), and (IV) are described wherein R^{a} is hydrogen, halo, hydroxyl, or alkyl, alkoxy, cycloalkyl, or arylalkoxy, each of which is optionally substituted. In another embodiment, compounds of formulae (II), (III), and (IV) are described wherein R^{a} is hydrogen, halo, hydroxyl, alkyl, alkoxy, cycloalkyl, or arylalkoxy. In another embodiment, compounds of formulae (II), (III), and (IV) are described wherein R^{a} is at C-7. In another embodiment, compounds of formulae (II), (III), and (IV) are described wherein R^{a} is at C-8. In another embodiment, compounds of formulae (II), (III), and (IV) are described wherein R^{a} is at C-9.

In another embodiment, compounds of formulae (II), (III), and (IV) and each of the foregoing embodiments are described wherein R^{c} is hydrogen. In another embodiment, compounds of formulae (II), (III), and (IV) and each of the foregoing embodiments are described wherein R^{c} is optionally substituted alkyl. In another embodiment, compounds of formulae (II), (III), and (IV) and each of the foregoing embodiments are described wherein R^{c} is alkyl. In another embodiment, compounds of formulae (II), (III), and (IV) and each of the foregoing embodiments are described wherein R^{c} is at C-5.

In another embodiment, compounds of formulae (II), (III), and (IV) and each of the foregoing embodiments are described wherein R^{d} is hydrogen.

In another embodiment, compounds of formulae (II), (III), and (IV) and each of the foregoing embodiments are described wherein R^{N} is independently selected in each instance from hydrogen, or alkyl, cycloalkyl, or arylalkyl, each of which is optionally substituted. In another embodiment, compounds of formulae (II), (III), and (IV) and each of the foregoing embodiments are described wherein R^{N} is independently selected in each instance from hydrogen, or alkyl, cycloalkyl, or arylalkyl, each of which is optionally substituted.

In another embodiment, there is provided metralindole, including the formula: 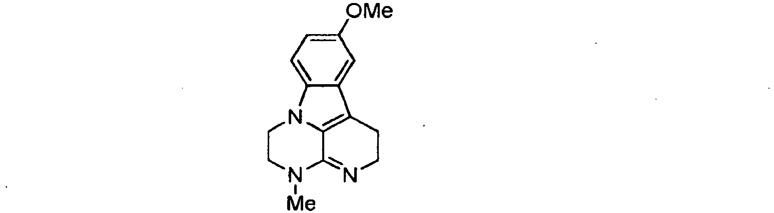 and pharmaceutically acceptable salts thereof, such as the hydrochloride salt.

In each of the foregoing and following embodiments, it is to be understood that the formulae include and represent not only all pharmaceutically acceptable salts of the compounds according to the first aspect of the invention, but also include any and all hydrates and/or solvates of the compound formulae. It is appreciated that certain functional groups, such as the hydroxy, amino, and like groups form complexes and/or coordination compounds with water and/or various solvents, in the various physical forms of the compounds. Accordingly, the above formulae are to be understood to include and represent those various hydrates and/or solvates. In each of the foregoing and following embodiments, it is also to be understood that the formulae include and represent each possible isomer, such as stereoisomers and geometric isomers, both individually and in any and all possible mixtures. In each of the foregoing and following embodiments, it is also to be understood that the formulae include and represent any and all crystalline forms, partially crystalline forms, and non crystalline and/or amorphous forms of the compounds.

The compounds described herein may be prepared according to conventional synthetic processes, including the process described by Gazengel et al., Journal of Heterocyclic Chemistry (1990), 27(7), 1947-51. For example, it is appreciated that the various analogs and derivatives of pirlindole itself described herein may be prepared by routine modification and optimization of the process described by Gazengel et al., such as by the appropriate selection of the corresponding starting materials used in the process.

In another embodiment, the compounds, or a pharmaceutically acceptable salt thereof is administered to a patient in need of relief at a dose in the range from about 0.1 mg/kg to about 10 mg/kg per day; from about 1 mg/kg to about 5 mg/kg per day, from about 1 mg/kg to about 4 mg/kg per day, from about 1 mg/kg to about 3 mg/kg per day, from about 1 mg/kg to about 2 mg/kg per day, from about 1.5 mg/kg to about 5 mg/kg per day, from about 1.5 mg/kg to about 4 mg/kg per day, from about 1.5 mg/kg to about 3 mg/kg per day, from about 1.5 mg/kg to about 2 mg/kg per day, from about 2 mg/kg to about 5 mg/kg per day, from about 2 mg/kg to about 4 mg/kg per day, or from about 2 mg/kg to about 3 mg/kg per day.

In another embodiment, the tetracyclic pyrazinoindole, or a pharmaceutically acceptable salt thereof is administered to a patient in need of relief at a daily dose in the range from about 1 mg to about 1,000 mg per day; from about 5 mg to about 600 mg per day, from about 5 mg to about 500 mg per day, from about 10 mg to about 400 mg per day, from about 50 mg to about 300 mg per day, from about 50 mg to about 250 mg per day, from about 50 mg to about 200 mg per day, from about 50 mg to about 150 mg per day, from about 50 mg to about 100 mg per day, from about 100 mg to about 300 mg per day, from about 200 mg to about 300 mg per day, or from about 150 mg to about 300 mg per day, or from about 150 mg to about 200 mg per day. It is appreciated that the foregoing doses may be administered to adults and/or teens, and that the corresponding doses administered to preteens, toddlers, or infants, are lower, such as illustratively by a factor of about 2, about 5, or about 10, respectively.

Each of the foregoing may be illustratively administered q.d., b.i.d., t.i.d, or by other conventional dosing protocols, including intermittent dosing protocols that have an off period. In addition, it is to be understood that at each dosing interval, the amount of the dose may be single or divided into various unit dosage forms. In another illustrative embodiment, the daily dose is administered t.i.d.

In another embodiment the methods described herein include a titration step where the dose is gradually increased over a predetermined time period, such as a two step protocol for adults as follows: 0.5 mg/kg thrice daily for 7 days, then 1 mg/kg thrice daily; or 0.75 mg/kg thrice daily for 7 days, then 1 mg/kg thrice daily.

In another embodiment the methods described herein include a titration step where the dose is gradually increased over a predetermined time period, such as a three step protocol for adults as follows: 0.25 mg/kg thrice daily for 7 days, then 0.5 mg/kg thrice daily for 7 days, then 1 mg/kg thrice daily; or 0.5 mg/kg thrice daily for 7 days, then 0.75 mg/kg thrice daily for 7 days, then 1 mg/kg thrice daily.

It is appreciated that the foregoing tritrating dosing protocols may be administered to adults and/or teens, and that the corresponding doses administered to preteens, toddlers, or infants, are lower, such as illustratively by a factor of about 2, about 5, or about 10, respectively, and may accordingly be based on the weight of the patient as indicated.

Optimal dosages and dosage regimens to be administered may be readily determined by routine experimentation, and it is understood that such optimal dosages and dosage regimens will vary with the mode of administration, the strength of the preparation and the advancement of the disease condition. In addition, factors associated with the particular patient being treated, including patient's sex, age, weight, diet, physical activity, time of administration and concomitant diseases, will result in the need to adjust dosages and/or regimens.

Without limiting the foregoing, it is appreciated that such lower doses of tetracyclic pyrazinoindoles may be more applicable to an ongoing, or chronic therapy, designed for continuous administration, rather than intermittent or acute administration. Accordingly, the daily dose may be divided and administered b.i.d. and/or t.i.d, although it is to be understood that q.d. dosing is described herein. It is to be understood that the illustrative doses described herein represent daily doses, and may be therefore administered q.d., b.i.d., t.i.d., and according to additional dosing protocols. In addition, it is to be understood that the doses may be single or divided.

As used herein the term "multiple sclerosis" or MS includes neurological disorders characterized by demyelination, including neurological disorders characterized by an autoimmune reaction leading to demyelination. It is understood that MS may cause numerous physical and mental symptoms, and often progresses to physical and cognitive disability. Disease onset usually occurs in young adults, and MS has been reported to be more common in women. MS has been reported to have a prevalence that ranges between 2 and 150 per 100,000 depending on the country or specific population (see, e.g., Rosati G (April 2001) Neurol. Sci. 22 (2): 117-39. PMID 1160361426).

MS affects the areas of the brain and spinal cord known as the white matter. White matter cells carry signals between the grey matter areas, where the processing is done, and the rest of the body. MS results in a thinning or complete loss of myelin and in an axonal damage. When the myelin is lost, the neurons can no longer effectively conduct neural impulses. More specifically, the destruction of oligodendrocytes, glial cells that are the cells responsible for creating and maintaining the neuronal myelin sheath, is generally accompanied by MS. Without being bound by theory, it is believed herein that either MS causes the destruction of oligodendrocytes by causing mitochondrial permeability transition pores to open, or that as a consequence of demyelination by another mechanism, mitochondrial permeability transition pores open, leading to the ultimate death of the oligodendrocytes.

MS takes several forms, with new symptoms occurring either in discrete attacks (relapsing forms) or slowly accumulating over time (progressive forms). Most people are first diagnosed with relapsing-remitting MS but develop secondary-progressive MS (SPMS) after a number of years. Between attacks, symptoms may go away completely, but permanent neurological problems often persist, especially as the disease advances.

Although much is known about the mechanisms involved in the disease process, the underlying cause or triggering of MS remains unknown. A well-reported theory is that the condition is autoimmune related. However, it has also been reported on the one hand that the disease is a metabolically dependent disease while on the other hand that it is caused by a virus such as Epstein-Barr. Still other theories have been reported, including that based on its virtual absence from tropical areas, MS may arise from a deficiency of vitamin D during childhood. Regardless of its cause, there is not current cure for MS, but several therapies have proven helpful. In each case, the treatments attempt to return function after an attack, prevent new attacks, and/or prevent worsening or progression of the disease leading to disability. The prognosis for successful therapy depends on the subtype of the disease, the individual patient's disease characteristics, the initial symptoms, and the degree of disability the person experiences as time advances.

Methods are described herein for treating primary progressive multiple sclerosis and/or secondary progressive multiple sclerosis and/or relapsing-remitting multiple sclerosis by administering one or more tetracyclic pyrazinoindoles, and/or pharmaceutically acceptable salts thereof, in an amount therapeutically effective to stabilize mitochondria.

Methods are described herein for treating primary progressive multiple sclerosis and/or secondary progressive multiple sclerosis and/or relapsing-remitting multiple sclerosis by administering one or more tetracyclic pyrazinoindoles, and/or pharmaceutically acceptable salts thereof, in an amount therapeutically effective to inhibit lipid peroxidation, and as a consequence, removing intracellular peroxides.

Methods are described herein for treating primary progressive multiple sclerosis and/or secondary progressive multiple sclerosis and/or relapsing-remitting multiple sclerosis by administering one or more tetracyclic pyrazinoindoles, and/or pharmaceutically acceptable salts thereof, in an amount therapeutically effective to protect cell membranes under hypoxia.

Methods are described herein for treating primary progressive multiple sclerosis and/or secondary progressive multiple sclerosis and/or relapsing-remitting multiple sclerosis by administering one or more tetracyclic pyrazinoindoles, and/or pharmaceutically acceptable salts thereof, in an amount therapeutically effective to augment the activity of Superoxide Dismutase in the brain.

Methods are described herein for treating primary progressive multiple sclerosis and/or secondary progressive multiple sclerosis and/or relapsing-remitting multiple sclerosis by administering one or more tetracyclic pyrazinoindoles, and/or pharmaceutically acceptable salts thereof, in an amount therapeutically effective to inhibit iron-mediated toxicity in nerve cells.

In another embodiment, the one or more compounds according to the first aspect of the invention are included in the compositions and methods in amounts therapeutically effective to (a) stabilize mitochondria and/or (b) inhibit lipid peroxidation. In one alternative aspect not of the invention, methods are described for treating one or more forms of MS by administering one or more tetracyclic pyrazinoindoles in therapeutically effective amounts to inhibit lipid peroxidation and stabilize mitochondria. In another alternative aspect not of the invention, methods are described for treating one or more forms of MS by administering one or more tetracyclic pyrazinoindoles in therapeutically effective amounts to inhibit lipid peroxidation. Without being bound by theory, it is believed herein that the compounds, compositions, and methods described herein for treating one or more forms of MS may be efficacious at least in part because the one or more tetracyclic pyrazinoindoles included therein are in therapeutically effective amounts to (a) stabilize mitochondria, (b) inhibit lipid peroxidation, (c) protect cell membranes under hypoxia, (d) augment the activity of superoxide dismutase in the brain, and/or (e) inhibit iron-mediated toxicity in nerve cells.

Without being bound by theory, it is believed herein that lipid peroxidation may be one of the mechanisms by which tetracyclic pyrazinoindoles are efficacious in treating MS. Generally, lipid peroxidation refers to the oxidative degradation of lipids. It is the process whereby free radicals remove electrons from the lipids in cell membranes, which may result in cell damage. This process proceeds by a free radical chain reaction mechanism. The process has been reported to most often affect polyunsaturated fatty acids because they contain multiple double bonds, and the intervening methylene (-CH₂-) groups possess relatively reactive hydrogen atoms. As with any radical reaction the reaction consists of three major steps: initiation, propagation and termination. Initiation is the step whereby a fatty acid radical is produced. The initiators in living cells are most notably reactive oxygen species (ROS), such as hydroxide radical (OH·), which combines with a hydrogen radical to form water and a resultant fatty acid radical. The fatty acid radicals are relatively unstable molecules, and readily react with molecular oxygen to form peroxyl-fatty acid radicals. Such peroxyl-fatty acid radicals are also relatively unstable and subsequently react with another free fatty acids to produce a numerous other fatty acid radicals and a lipid peroxides, including cyclic peroxides when the fatty acid radicals react intramolecularly. This radical reaction propagation system continues in this chain reaction mechanism. The radical reaction stops when two radicals react and produce a non-radical species in a termination step. This step happens only when the concentration of radical species is sufficiently high for there to be a high probability that two radicals will collide. Living organisms have evolved different molecules that speed up termination by trapping free radicals and terminating chain reaction mechanism thereby protecting the cell membrane. One important actor in the termination or quenching of radical compounds is the antioxidant vitamin E. Other anti-oxidants made within the body include the enzymes superoxide dismutase, catalase, and peroxidase.

It has been reported that CNS myelin is highly susceptible to free radical attack (Konat et al., Effect of reactive oxygen species on myelin membrane proteins. J. Neurochem; 45:1113-1118 (1985); Vargas et al., Evidence that oxidative stress is increased in patients with X-linked adrenoleukodystrophy. Biochim Biophys Acta. 1688(1):26-32 (2004 Jan 20); Ferretti et al., Increased levels of lipid hydroperoxides in plasma of patients with multiple sclerosis: a relationship with paraoxonase activity. sclerosis. Mult Scler. 11(6):677-82 (Dec 2005); Gonsette, Neurodegeneration in multiple sclerosis: The role of oxidative stress and excitotoxicity. J Neurol Sci. 274(1-2):48-53 (2008 Nov 15); Bruhwyler et al., Pirlindole: a selective reversible inhibitor of monoamine oxidase A. A review of its preclinical properties. Pharmacol Res. 36(1):23-33 (1997 Jul)). Evidence that lipid peroxidation is involved in the development of MS has been reported (Naidoo & Knapp, Studies of Lipid Peroxidation Products in Cerebrospinal Fluid and Serum in Multiple Sclerosis and Other Conditions. Clinical Chemistry 38/12:2449-2454 (1992)). Other evidence of the involvement of lipid peroxidation includes the observation of increased osmotic and mechanical fragility of MS erythrocytes, abnormal glutathione peroxidase activity in MS erythrocytes, decreased proportions of the phospholipid classes that have the highest polyunsaturated fatty acid content in MS myelin and other tissues, and increased incidence of MS in populations consuming high proportions of animal fats, which may be deficient in vitamin E.

However, without being bound by theory, it is believed herein that the therapeutic potential in MS of a treatment using a tetracyclic pyrazinoindole is not limited to the observed activity of those compounds against lipid peroxidation, or alternatively that other compounds capable of lipid peroxidation intervention would be efficacious. For example, a well known chemotherapy agent etoposide has been reported to possess lipid antioxidant activity. However, long term therapy using etoposide has been reportedly limited due to the strong induction of immunosuppression and accompanying toxicity.

Without being bound by theory, it is also believed herein that the success of the methods using a tetracyclic pyrazinoindole may be due at least in part to the particular pharmacokinetic characteristics and blood-brain-barrier permeability, in conjunction with the particular adverse event profile, shown by those compounds. Those abilities are contrary to what has been observed with other lipid antioxidants, which may have an unacceptable adverse event profile, such as has been observed with etoposide, unfavorable pharmacokinetic characteristics, such as exhibited by PNU-87663, or reduced efficacy, such as has been observed with tirilazad mesylate.

As used herein, the term "dimebolin" generally refers to hydrogenated pyrido[4,3-b]indoles according to formulae (A) - (E) and pharmaceutically acceptable salts of the foregoing. It is also to be understood that in each of the foregoing, any corresponding pharmaceutically acceptable salt is also included in the illustrative embodiments described herein. One such dimebolin is Dimebon, a known antihistamine drug that has been used clinically for many years, and has recently shown potential in the treatment of Alzheimer's disease (see, e.g., Doody et al., Lancet 2008;372: 207-215; Bachurin et al., Annals of the New York Academy of Sciences. 2001;939: 425-435). Each of the foregoing publications, and each additional publication cited herein, is incorporated herein in its entirety by reference. Additional dimebolins are described in PCT international application Serial No. PCT/US2009/060557.

In addition, described herein are other illustrative dimebolins of formulae (A), (B), (C), (D), and (E). The formulae include various functional groups on aromatic rings, such as R³. It is to be understood that derivatives of those compounds also include the compounds having for example different functional groups on those aromatic rings than those explicitly set forth in the definition of formulae (A), (B), (C), (D), and (E). In addition, it is to be understood that derivatives of those compounds also include the compounds having those same or different functional groups at different positions on the aromatic ring. Similarly, derivatives include parallel variations of other functional groups on the compounds described herein, such as R¹, and the like. Illustrative derivatives include, but are not limited to, both those compounds that may be synthetically prepared from the compounds described herein, as well as those compounds that may be prepared in a similar way as those described herein, but differing in the selection of starting materials.

In addition, as used herein the term dimebolins also refers to analogs of the compounds described herein. For example, illustrative analogs include, but are not limited to, those compounds that share functional and in some cases structural similarity to those compounds described herein. For example, described herein are illustrative dimebolins of formulae (A), (B), and (C) that include a 2,3,4,5-tetrahydro-1H-pyridoindole ring system. Illustrative analogs include, but are not limited to, the corresponding ring expanded compounds, such as the corresponding azepinoindole ring system, and the like. Other illustrative analogs include, but are not limited to, the corresponding ring systems that include additional heteroatoms, such as the corresponding pyridazinoindole ring system, and the like. Therefore, it is to be understood that all such compounds are also considered to be dimebolins,

In addition, as used herein the term dimebolins also refers to prodrug derivatives of the compounds described herein, and including prodrugs of the various analogs and derivatives thereof.

In another embodiment, dimebolins of formula (A) 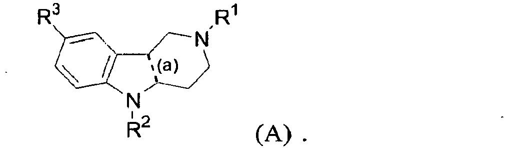 or a pharmaceutically acceptable salt thereof are described, wherein R¹ is hydrogen, or alkyl, heteroalkyl, cycloalkyl, cycloheteroalkyl, aryl, heteroaryl, arylalkyl, or heteroarylalkyl, each of which is optionally substituted; or R¹ and the attached nitrogen form an amide, carbamate, or urea, or thiono derivative thereof or arylalkyl; R² is hydrogen, or alkyl or arylalkyl, each of which is optionally substituted; R³ is hydrogen, halo, or hydroxy, or alkoxy, acyloxy, alkyl, heteroalkyl, cycloalkyl, cycloheteroalkyl, aryl, heteroaryl, arylalkyl, or heteroarylalkyl, each of which is optionally substituted; and bond (a) is a single bond or a double bond.

In another embodiment, dimebolins of formula (A) are described wherein wherein R¹ is alkyl or arylalkyl; R² is hydrogen, benzyl, or 6-methylpyridinyl-3-ethyl; R³ is hydrogen, alkyl, or halo; and bond (a) is a single bond or a double bond.

In another embodiment, dimebolins of formula (A) wherein R¹ is methyl, ethyl or benzyl are described. In another embodiment, dimebolins of formula (A) wherein R² is hydrogen, benzyl, or 6-methylpyridinyl-3-ethyl are described. In another embodiment, dimebolins of formula (A) wherein R³ is hydrogen, methyl, or bromo are described. In another embodiment, dimebolins of formula (A) wherein bond (a) is a single bond; R¹ and R³ are each methyl; and R² is hydrogen are described. In another embodiment, dimebolins of formula (A) wherein bond (a) is a single bond; and the ring fusion is cis are described. In another embodiment, dimebolin of formula (A) wherein bond (a) is a double bond; R¹ is ethyl or benzyl; and R² and R³ are each hydrogen; or R¹ and R³ are each methyl; and R² is benzyl; or R¹ is methyl; R² is 6-methylpyridinyl-3-ethyl; and R³ is hydrogen; or R¹ and R³ are each methyl; and R² is 6-methylpyridinyl-3-ethyl; or R¹ is methyl; R² is hydrogen; and R³ is hydrogen or methyl; or R¹ is methyl; R² is hydrogen; and R³ is bromo are described.

In another embodiment, dimebolin of formula (A) where R¹ is selected from the group consisting of alkyl, lower alkyl and arylalkyl, R² is selected from the group consisting of hydrogen, arylalkyl and substituted heteroarylalkyl; and R³ is selected from the group consisting of hydrogen, alkyl, lower alkyl and halo are described.

In one variation, R¹ is alkyl, such as an alkyl selected from the group consisting of C₁-C₁₅alkyl, C₁₀-C₁₅alkyl, C₁-C₁₀alkyl, C₂-C₁₅alkyl, C₂-C₁₀alkyl, C₂-C₈alkyl, C₄-C₈alkyl, C₆-C₈alkyl, C₆-C₁₅alkyl, C₁₅-C₂₀alkyl; C₁-C₈alkyl and C₁-C₆alkyl. In another variation, R¹ is arylalkyl. In another variation, R¹ is lower alkyl, such as a lower alkyl selected from the group consisting of C₁-C₂-alkyl, C₁-C₄alkyl, C₂-C₄alkyl, C₁-C₅alkyl, C₁-C₃alkyl, and C₂-C₅alkyl. In another variation, R¹ is a straight chain alkyl group. In another variation, R¹ is a branched alkyl group. In another variation, R¹ is a cyclic alkyl group. In another variation, R¹ is methyl. In another variation, R¹ is ethyl. In another variation, R¹ is methyl or ethyl. In another variation, R¹ is methyl or an arylalkyl group such as benzyl. In another variation, R¹ is ethyl or an arylalkyl group such as benzyl. In another variation, R¹ is an arylalkyl group. In another variation, R¹ is an arylalkyl group where any one of the alkyl or lower alkyl substituents listed in the preceding paragraphs is further substituted with an aryl group (e.g., Ar-C₁-C₆ alkyl, Ar-C₁-C₃ alkyl or Ar-C₁-C₁₅ alkyl). In another variation, R¹ is an arylalkyl group where any one of the alkyl or lower alkyl substituents listed in the preceding paragraphs is substituted with a single ring aryl residue. In another variation, R¹ is an arylalkyl group where any one of the alkyl or lower alkyl substituents listed in the preceding paragraphs is further substituted with a phenyl group (e.g. , Ph-C₁-C₆alkyl or Ph-C₁-C₃alkyl, Ph-C₁-C₁₅alkyl). In another variation, R¹ is benzyl. All of the variations for R¹ are to be understood to be described and to be combined with any of the variations stated below for R² and R³ the same as if each and every combination of R¹, R² and R³ were specifically and individually listed.

In another variation, R² is H. In another variation, R² is an arylalkyl group. In another variation, R² is a substituted heteroarylalkyl group. In another variation, R² is hydrogen or an arylalkyl group. In another variation, R² is hydrogen or a substituted heteroarylalkyl group. In another variation, R² is an arylalkyl group or a substituted heteroarylalkyl group. In another variation, R² is selected from the group consisting of hydrogen, an arylalkyl group and a substituted heteroarylalkyl group. In another variation, R² is an arylalkyl group where R² can be any one of the arylalkyl groups noted for R¹ above, the same as if each and every arylalkyl variation listed for R¹ is separately and individually listed for R². In another variations, R² is a substituted heteroarylalkyl group, where the alkyl moiety of the heteroarylalkyl can be any alkyl or lower alkyl group, such as those listed above for R¹. In another variation, R² is a substituted heteroarylalkyl where the heteroaryl group is substituted with 1 to 3 C₁-C₃ alkyl substituents (e.g., 6-methyl-3-pyridylethyl). In another variation, R² is a substituted heteroarylalkyl group wherein the heteroaryl group is substituted with 1 to 3 methyl groups. In another variation, R² is a substituted heteroarylalkyl group wherein the heteroaryl group is substituted with one lower alkyl substituent. In another variation, R² is a substituted heteroarylalkyl group wherein the heteroaryl group is substituted with one C₁-C₃ alkyl substituent. In one variation, R² is a substituted heteroarylalkyl group wherein the heteroaryl group is substituted with one or two methyl groups. In another variation, R² is a substituted heteroarylalkyl group wherein the heteroaryl group is substituted with one methyl group.

In other variations, R² is any one of the substituted heteroarylalkyl groups in the immediately preceding paragraph where the heteroaryl moiety of the heteroarylalkyl group is a single ring heteroaryl group. In other variations, R² is any one of the substituted heteroarylalkyl groups in the immediately preceding paragraph where the heteroaryl moiety of the heteroarylalkyl group is a multiple condensed ring heteroaryl group. In other variations, R² is any one of the substituted heteroarylalkyl groups in the immediately preceding paragraph where the heteroarylalkyl moiety is a pyridyl group (Py). In one variation, R² is 6-CH₃-3-Py-(CH₂)₂. An example of a compound containing this moiety is dimebon.

In another variation, R³ is hydrogen. In other variations, R³ is any one of the alkyl groups noted for R¹ above, the same as if each and every alkyl variation listed for R¹ is separately and individually listed for R . In another variation, R³ is a halo group. In another variation, R³ is hydrogen or an alkyl group. In another variation, R³ is a halo or alkyl group. In another variation, R³ is hydrogen or a halo group. In another variation, R³ is selected from the group consisting of hydrogen, alkyl and halo. In another variation, R³ is Br. In another variation, R³ is I. In another variation, R³ is F. In another variation, R³ is Cl.

In another variation, the compound is of the Formula (A) and R¹ is selected from a lower alkyl or benzyl; R² is selected from a hydrogen, benzyl or 6-CH₃-3-Py-(CH₂)₂ and R is selected from hydrogen, lower alkyl or halo, or any pharmaceutically acceptable salt thereof. In another variation, R¹ is selected from CH₃, CH₃CH₂, or benzyl; R² is selected from H, benzyl, or 6-CH₃-3-Py-(CH₂)₂; and R³ is selected from H, CH₃ or -Br, or any pharmaceutically acceptable salt thereof. In another variation the compound is selected from the group consisting of: cis(±)2,8-dimethyl-2,3,4,4a,5,9b-hexahydro-1H-pyrido[4,3-b]indole as a racemic mixture or in the substantially pure (+) or substantially pure (-) form; 2-ethyl- 2,3,4,5-tetrahydro-1H-pyrido[4,3-b]indole; 2-benzyl-2,3,4,5-tetrahydro-1H-pyrido[4,3-b]indole; 2,8-dimethyl-5-benzyl-2,3,4,5-tetrahydro-1H-pyrido[4,3-b]indole; 2-methyl-5-(2- methyl-3-pyridyl)ethyl-2,3,4,5-tetrahydro-1H-pyrido[4,3-b]indole; 2,8-dimethyl-5-(2-(6- methyl-3-pyridyl)ethyl)-2,3,4,5-tetrahydro-1H-pyrido[4,3-b]indole; 2-methyl-2,3,4,5- tetrahydro-1H-pyrido[4,3-b]indole; 2,8-dimethyl-2,3,4,5-tetrahydro-1H-pyrido[4,3-b]indole; or 2-methyl-8-bromo-2,3,4,5-tetrahydro-1H-pyrido[4,3-b] indole or any pharmaceutically acceptable salt of any of the foregoing.

In another variation, the compound is of the formula (A) wherein R¹ is CH₃, R² is H and R³ is -CH₃ or any pharmaceutically acceptable salt thereof. The compound may be of the Formula (A) where R¹ is CH₃CH₂ or benzyl, R² is H, and R³ is CH₃ or any pharmaceutically acceptable salt thereof. The compound may be of the Formula (A) where R¹ is CH₃, R² is benzyl, and R³ is -CH₃ or any pharmaceutically acceptable salt thereof. The compound may be of the Formula (A) where R¹ is -CH₃, R² is 6-CH₃-3-Py-(CH₂)₂-, and R³ is -H or any pharmaceutically acceptable salt thereof. The compound may be of the Formula (A) where R² is 6-CH₃-3-Py-(CH₂)₂- or any pharmaceutically acceptable salt thereof. The compound may be of the Formula (A) where R¹ is -CH₃, R² is -H, and R³ is -H or -CH₃ or any pharmaceutically acceptable salt, thereof. The compound may be of the Formula (A) where R¹ is -CH₃, R² is -H, and R³ is -Br, or any pharmaceutically acceptable salt thereof. The compound may be of the Formula (A) where R¹ is selected from a lower alkyl or arylalkyl, R² is selected from a hydrogen, arylalkyl or substituted heteroarylalkyl and R³ is selected from hydrogen, lower alkyl or halo.

In another embodiment, dimebolins of formula (B) 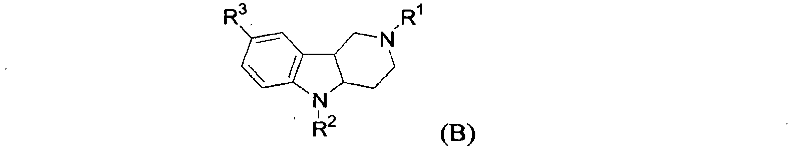 or a pharmaceutically acceptable salt thereof are described, wherein R¹ is alkyl or arylalkyl; R² is hydrogen, benzyl, or 6-methylpyridinyl-3-ethyl; R³ is hydrogen, alkyl, or halo; and bond (a) is a single bond or a double bond.

In another embodiment, dimebolins of formula (B) are described wherein R¹ is methyl, ethyl or benzyl. In another embodiment, dimebolins of formula (B) are described wherein R² is hydrogen, benzyl, or 6-methylpyridinyl-3-ethyl. In another embodiment, dimebolins of formula (B) are described wherein R³ is hydrogen, methyl, or bromo. In another embodiment, dimebolins of formula (B) are described wherein R¹ and R³ are each methyl; and R² is hydrogen. In another embodiment, dimebolins of formula (B) are described wherein the ring fusion is cis. In another embodiment, dimebolins of formula (B) are described in a pharmaceutically acceptable quaternary salt form.

In another embodiment, dimebolins of formula (C) 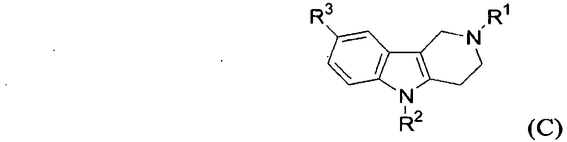 or a pharmaceutically acceptable salt thereof are described, wherein R¹ is alkyl or arylalkyl; R² is hydrogen, benzyl, or 6-methylpyridinyl-3-ethyl; R³ is hydrogen, alkyl, or halo; and bond (a) is a single bond or a double bond.

In another embodiment, dimebolins of formula (C) are described wherein R¹ is methyl, ethyl or benzyl. In another embodiment, dimebolins of formula (C) are described wherein R² is hydrogen, benzyl, or 6-methylpyridinyl-3-ethyl. In another embodiment, dimebolins of formula (C) are described wherein R³ is hydrogen, methyl, or bromo.

In another embodiment, dimebolins of formula (C) are described wherein R¹ is ethyl or benzyl; and R² and R³ are each hydrogen; or R¹ and R³ are each methyl; and R² is benzyl; or R¹ is methyl; R² is 6-methylpyridinyl-3-ethyl; and R³ is hydrogen; or R¹ and R³ are each methyl; and R² is 6-methylpyridinyl-3-ethyl; or R¹ is methyl; R² is hydrogen; and R³ is hydrogen or methyl; or R¹ is methyl; R² is hydrogen; and R³ is bromo. In another embodiment, dimebolins of formula (C) are described in a pharmaceutically acceptable quaternary salt form.

Additional illustrative dimebolins that may included in the compositions and methods described herein include hydrogenated pyrido [4,3-b] indoles of the formula (D) or the formula (E): 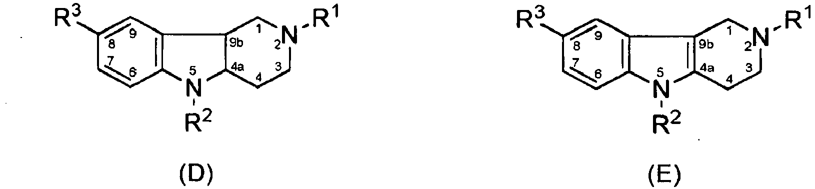 For compounds of a general formulae (D) or (E), R¹ represents CH₃, CH₃CH₂, or PhCH₂ (benzyl); R² is H, PhCH₂, or 6CH₃-3-Py-(CH₂)₂; R³ is H, CH₃, or Br, in any combination of the above substituents. All possible combinations of the substituents of formulae (D) or (E) are described herein as specific and individual compounds the same as if each single and individual compound were listed by chemical name. Also contemplated are the compounds of formulae (D) or (E), with any deletion of one or more possible moieties from the substituent groups listed above, such as for example where R¹ represents CH₃. In one variation, R² is H, PhCH₂, or 6CH₃-3-Py-(CH₂)₂; and R³ is H, CH₃, or Br, or where R¹ represents CH₃; R² is 6CH₃-3-Py-(CH₂)₂; and R³ represents H, CH₃, or Br.

The compound may be formula (D), where R¹ is CH₃, R² is H, and R³ is CH₃. The compound may be formula (E), where R¹ is represented by CH₃, CH₃CH₂, or PhCH₂; R² is H, PhCH₂, or 6CH₃-3-Py-(CH₂)₂; R³ is H, CH₃, or Br. The compound may be formula (E), where R¹ is CH₃CH₂ or PhCH₂, R² is H, and R³ is H; or a compound, where R¹ is CH₃, R² is PhCH₂, R³ is CH₃; or a compound, where R¹ is CH₃, R² is 6-CH₃-3-Py-(CH₂)₂, and R³ is CH₃; or a compound, where R¹ is CH₃, R² is H, R³ is H or CH₃; or a compound, where R¹ is CH₃, R² is H, R³ is Br.

In another embodiment, the compositions include a therapeutically effective amount of a dimebolin of the formula 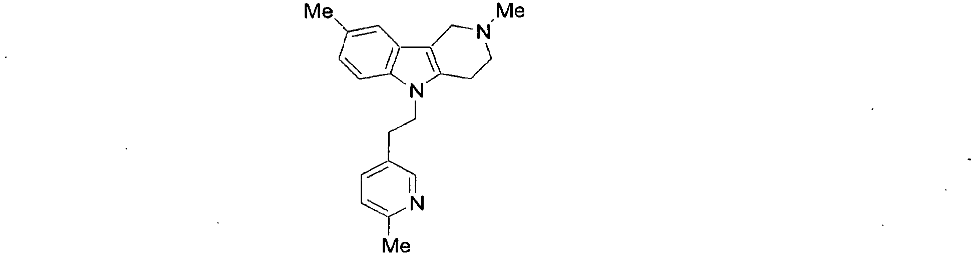 or a pharmaceutically acceptable salt, such as the hydrochloride salt.

Additional illustrative dimebolins that may included in the compositions and methods described herein include hydrogenated pyrido[4,3-b]indoles or pharmaceutically acceptable salts thereof, such as an acid or base salt thereof. A hydrogenated pyrido[4,3-b]indole can be a tetrahydropyrido[4,3-b]indole or pharmaceutically acceptable salt thereof. The hydrogenated pyrido[4,3-b]indole can also be a hexahydropyrido[4,3-b]indole or pharmaceutically acceptable salt thereof. The hydrogenated pyrido[4,3-b]indole compounds can be substituted with 1 to 3 substituents, although unsubstituted hydrogenated pyrido[4,3-b]indole compounds or hydrogenated pyrido[4,3-b]indole compounds with more than 3 substituents are also contemplated. Suitable substituents include but are not limited to alkyl, lower alkyl, arylalkyl, heteroarylalkyl, substituted heteroarylalkyl, substituted arylalkyl, and halo.

The hydrogenated pyrido[4,3-b]indoles can be in the form of pharmaceutically acceptable salts thereof. The pharmaceutically acceptable salts include pharmaceutically acceptable acid salts. Examples of particular pharmaceutically acceptable salts include hydrochloride salts or dihydrochloride salts. In a particular variation, the hydrogenated pyrido[4,3-b]indole is a pharmaceutically acceptable salt of 2,8-dimethyl-5-(2-(6-methyl-3-pyridyl)ethyl)-2,3,4,5-tetrahydro-1H-pyrido[4,3-b]indole, such as 2,8-dimethyl-5-(2-(6-methyl-3-pyridyl)ethyl)-2,3,4,5-tetrahydro- 1H-pyrido[4,3-b]indole dihydrochloride (dimebon).

In another embodiment, the dimebolins are selected from 2-methyl-2,3,4,5-tetrahydro-1H-pyrido[4,3-b]indole; 2,8-dimethyl-2,3,4,5-tetrahydro-1H-pyrido[4,3-b]indole, or its methyliodide; cis-(±) 2,8-dimethyl-2,3,4,4a,5,9b-hexahydro1H-pyrido[4,3-b]indole, or its dihydrochloride; 2-methyl-8-bromo-2,3,4,5-tetrahydro-1H-pyrido[4,3-b]indole, or its hydrochloride; 2-ethyl-2,3,4,5-tetrahydro-1H-pyrido[4,3-b]indole; 2-benzyl-2,3,4,5-tetrahydro-1H-pyrido[4,3-b]indole; 2,8-dimethyl-5-benzyl-2,3,4,5-tetrahydro-1H-pyrido[4,3-b]indole, or its hydrochloride; 2-methyl-5-[2-(6-methyl-3-pyridyl)ethyl]-2,3,4,5-tetrahydro-1H-pyrido[4,3-b] indole, or its sesquisulfate monohydrate; and 2,8-dimethyl-5-[2-(6-methyl-3-pyridyl)ethyl]-2,3,4,5-tetrahydro-1H-pyrido[4,3-b]indole, or its dihydrochloride.

Additional illustrative dimebolins that may included in the compositions and methods described herein include cis(±) 2,8-dimethyl-2,3,4,4a,5,9b-hexahydro-1H-pyrido[4,3-b]indole and its dihydrochloride ; 2-ethyl-2,3,4,5-tetrahydro-1H-pyrido[4,3-b]indole; 2-benzyl-2,3,4,5-tetrahydro-1H-pyrido[4,3-b]indole; 2,8-dimethyl-5-benzyl-2,3,4,5-tetrahydro-1H-pyrido[4,3-b]indole and its dihydrochloride; 2-methyl-5-(2-methyl-3-pyridyl)ethyl-2,3,4,5-tetrahydro-1H-pyrido[4,3-b]indole and its sesquisulfate; 2,8-dimethyl-5-(2-(6-methyl-3-pyridyl)ethyl)-2,3,4,5-tetrahydro-1H-pyrido[4,3-b]indole and its dihydrochloride (dimebon); 2-methyl-2,3,4,5-tetrahydro-1H-pyrido[4,3-b]indole; 2,8-dimethyl-2,3,4,5-tetrahydro-1H-pyrido[4,3-b]indole and its methyl iodide; 2-methyl-8-bromo-2,3,4,5-tetrahydro-1H-pyrido[4,3-b]indole and its hydrochloride.

In a another variation, the hydrogenated pyrido[4,3-b]indole is 2,8-dimethyl-5-(2-(6-methyl-3-pyridyl)ethyl)-2,3,4,5-tetrahydro-1H-pyrido[4,3-b]indole or a pharmaceutically acceptable salt thereof. The compound for use in the compositions may be 2,8-dimethyl-5-(2-(6- methyl-3-pyridyl)ethyl-2,3,4,5-tetrahydro-1H-pyrido[4,3-b]indole or any pharmaceutically acceptable salt thereof, such as an acid salt, a hydrochloride salt or a dihydrochloride salt thereof.

In each of the foregoing and following embodiments, it is to be understood that the formulae include and represent not only all pharmaceutically acceptable salts of the dimebolins, but also include any and all hydrates and/or solvates of the compound formulae. It is appreciated that certain functional groups, such as the hydroxy, amino, and like groups form complexes and/or coordination compounds with water and/or various solvents, in the various physical forms of the compounds. Accordingly, the above formulae are to be understood to include and represent those various hydrates and/or solvates. In each of the foregoing and following embodiments, it is also to be understood that the formulae include and represent each possible isomer, such as stereoisomers and geometric isomers, both individually and in any and all possible mixtures. In each of the foregoing and following embodiments, it is also to be understood that the formulae include and represent any and all crystalline forms, partially crystalline forms, and non crystalline and/or amorphous forms of the compounds.

Dimebolins may be prepared according to Horlein, Chem. Ber., 1954, Bd.87, hft 4, p. 463-472; Cattanach et al., J. Chem. Soc. (ser. C) 1968, 1235-1243; Yurovskaya and Rodionov, Khim. Geterots. Soed., 1981, No. 8, p. 1072-1078; Yakhontov and Glushkova, Synthatic Drugs (edited by A. G. Natradze), Moscow, "Meditsina Publishers", 1983, p. 234-237; Buu-Hoi et al., J. Chem. Soc., 1964, No. 2, p. 708-711; Kucherova and Kochetkov, J. Obshch. Khim., 1956, v. 26, p. 3149-3154; and Kost et al., "Khim. Geterots. Soed.", 1973, No. 2, p. 207-212; Yakhontov, L.N., Glushkov, R.G, Synthetic Therapeutic Drugs; A.G. Natradze, Ed., Moscow Medicina, 1983, p. 234-237; C.J. Cattanach, A. Cohen & B.H. Brown, J. Chem. Soc. (Ser. C) 1968, p. 1235-1243; N. P. Buu-Hoi, O. Roussel, P. Jacquignon, J. Chem. Soc, 1964, N 2, p. 708-711; N.F. Kucherova and N.K. Kochetkov (General Chemistry (Russ.), 1956, 26:3149- 3154); A.N. Kost, M.A. Yurovskaya, T.V. Mel'nikova, in Chemistry of Heterocyclic Compounds, 1973, N 2, p. 207-212 ; U, Horlein in Chem. Ber., 1954, Bd. 87, hft 4, 463-p. 472; and M. Yurovskaya and LL. Rodionov in Chemistry of Heterocyclic Compounds (1981, N 8, p. 1072-10).

It is to be understood that when bond (a) is a single bond, such dimebolins include two stereocenters in the pyrido[4,3-b]indole ring structure (e.g., carbons 4a and 9b of formula (A)), which can adopt a cis or a trans ring fusion. A composition may comprise such a compound in substantially pure form, such as a composition of substantially pure (S,S), (R,R), (S,R), or (R,S) compound. A composition of substantially pure compound generally means that the composition contains no more than 15% or no more than 10% or no more than 5% or no more than 3% or no more than 1% impurity of the compound in a different stereochemical form. For instance, a composition of substantially pure (S,S) compound generally means that the composition contains no more than 15% or no more than 10% or no more than 5% or no more than 3% or no more than 1% of the R,R or S,R or R,S form of the compound. A composition may contain the compound as mixtures of such stereoisomers, where the mixture may be enantiomers, for example (S,S) and (R,R), or diastereomers, for example (S,S) and (R,S) or (S,R), in equal or unequal amounts. A composition may contain the compound as a mixture of 2 or 3 or 4 such stereoisomers in any ratio of stereoisomers. Compounds disclosed herein having stereocenters other than in the pyrido[4,3-b]indole ring structure intends all stereochemical variations of such compounds, including but not limited to enantiomers and diastereomers in any ratio, and includes racemic and enantioenriched and other possible mixtures. Unless stereochemistry is explicitly indicated in a structure, the structure is intended to embrace all possible stereoisomers of the compound depicted, either alone or as mixtures.

In another embodiment, compositions and methods are described herein that also include one or more additional NMDA antagonists. Illustratively, the additional NMDA antagonist is an uncompetitive channel blocker. Illustrative uncompetitive channel blockers include, but are not limited to, riluzole, memantine, amantadine, dextromethorphan, dextrorphan, ibogaine, ketamine, phencyclidine, tiletamine, remacemide, and the like, and pharmaceutically acceptable salts thereof. In one variation, the additional NMDA receptor antagonists are selected from riluzole, memantine, amantadine, and dextromethorphan, and pharmaceutically acceptable salts thereof. In another variation, the additional NMDA receptor antagonist is memantine (also known as AXURA, AKATINOL, NAMENDA, EBIXA, and 1-amino-3,5-dimethylada-mantane), or a pharmaceutically acceptable salts thereof. Illustratively, the additional NMDA antagonist is a noncompetitive antagonist. Illustrative noncompetitive antagonists include, but are not limited to, HU-211, Dizocilpine (MK-801), aptiganel (CERESTAT, CNS-1102), remacemide, and the like, and pharmaceutically acceptable salts thereof. Illustratively, the additional NMDA antagonist is a glycine antagonist, such as a compound that binds to and/or operates through an allosteric site of action at the glycine binding site. Illustrative glycine antagonists include, but are not limited to, 7-chlorokynurenate, 5,7-dichlorokynurenic acid (DCKA), kynurenic acid, 1-aminocyclopropanecarboxylic acid (ACPC), and the like, and pharmaceutically acceptable salts thereof.

In another embodiment, one or more compounds according to the first aspect of the invention are co-administered with another NMDA antagonist, such as with amantadine. In another embodiment, one or more compounds according to the first aspect of the invention are co-administered with another NMDA antagonist, such as with memantine. In another embodiment, one or more compounds according to the first aspect of the invention are co-administered with another NMDA antagonist, such as with HU-211.

In another embodiment, one or more compounds according to the first aspect of the invention are co-administered with another NMDA antagonist, such as a competitive antagonist, including but not limited to 2-amino-7-phosphonoheptanoic acid (AP7), R-2-amino-5-phosphonopentanoate (APV), and CPPene (3-[(R)-2-carboxypiperazin-4-yl]-prop-2-enyl-1-phosphonic acid).

Without being bound by theory, it is believed herein that anti-excitotoxicity action is beneficial to the methods described herein. It is believed that compounds according to the first aspect of the invention may also exhibit an anti-excitotoxicity effect. Accordingly, the co-administration of one or more NMDA receptor antagonists capable of further reducing cell damage, such as may be caused by lipid destruction or excitotoxicity, may be beneficial as a cotherapy with the administration of pirlindoles as described herein.

Without being bound by theory, it has also been discovered that glutamate receptors may play an important role in the axonal injury and neuronal cell death that is observed in MS. In particular, over-stimulation of NMDA receptors has been reported to lead to excessive mitochondrial calcium accumulation and damage, which is a critical step in excitotoxic death. Nevertheless, it is appreciated that blockade of all NMDA sites may be accompanied by several side effects, such as has been observed with PCP narcotics, and other non-selective NMDA antagonists. Such non-selective blockage is advantageously avoided by the selective blockade of certain NMDA sites. It is therefore understood that activity at selective and/or specific NMDA subunits may be advantageous in the treatment of MS, and it is appreciated that compounds described herein may have such selective and/or specific activity.

In another embodiment not of the invention, methods are described herein for treating multiple sclerosis that include the step of co-administering a therapeutically effective amount of one or more tetracyclic pyrazinoindoles described herein to a patient with one or more inhibitors of HMG-CoA reductase, also referred to as statins. In one variation, methods are described herein for treating Primary Progressive Multiple Sclerosis. In another variation, methods are described herein for treating Secondary Progressive Multiple Sclerosis.

In another embodiment, compositions are described herein that also include one or more HMG-CoA reductase inhibitors. Illustrative HMG-CoA reductase inhibitors include, but are not limited to, simvastatin, lovastatin, pravastatin, fluvastatin, atorvastatin, rosuvastatin, cerivastatin, and the like, and pharmaceutically acceptable salts thereof. In one variation, the HMG-CoA reductase inhibitor is simvastatin and/or lovastatin, or a pharmaceutically acceptable salt thereof. Without being bound by theory, it is believed herein that statins may exhibit NMDA receptor antagonist activity. Accordingly, the co-administration of one or more statins capable of further reducing cell damage, such as may be caused by lipid destruction or excitotoxicity, may be beneficial as a cotherapy with the administration of pirlindoles as described herein.

Statins have recently been reported to induce biochemical changes that can be beneficial to patients suffering from multiple sclerosis. In particular it has been reported that in addition to lowering cholesterol levels, statins have additional properties such as endothelial protection via actions on the nitric oxide synthetase system as well as antioxidant, anti-inflammatory and antiplatelet effects. Illustrative statins include simvastatin, pravastatin, lovastatin, fluvastatin, atorvastatin, rosuvastatin or cerivastatin, or a therapeutically active acid addition salt form of any of the foregoing

In another embodiment of the methods described herein not of the invention, a therapeutically effective amount of an antipsychotic compound or an atypical antipsychotic compound, or an analog or derivative thereof, or a pharmaceutically acceptable salt of the foregoing, is co-administered with one or more tetracyclic pyrazinoindole described herein to a patient in need of relief or suffering from multiple sclerosis. Illustrative antipsychotic compounds include, but are not limited to, tricyclic antidepressants. Illustrative atypical antipsychotics include, but are not limited to, risperidone, olanzapine, clozapine, quetiapane, aripiprazole, amisulpiride, ziprasidone

Illustrative compounds that are co-administered in the methods described herein include compounds of the formula 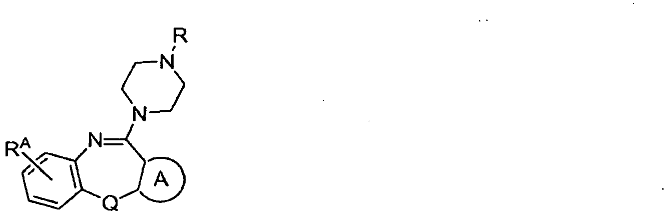 where R is alkyl or substituted alkyl, such as C1-C4 alkyl, alkoxyalkyl, hydroxyalkoxyalkyl, and the like; R^{A} is hydrogen or represents from 1 to 4 substituents, such as alkyl, halo, amino, alkoxy, and the like; Q is NH, O, or S; and A represents an optionally substituted fused aromatic ring, such as benzo, furano, thieno, thiazolo, oxazolo and imidazolo.

In another embodiment, Q is NH; A is optionally substituted benzo, or optionally substituted five or six membered aromatic ring having one to three hetero atoms independently selected from N, O, and S; R is hydrogen or (C₁₋₄) alkyl optionally substituted with OH, OR³, or OCH₂CH₂OH; where R³ is (C₁₋₂) alkyl; and R^{A} is hydrogen, or one or two substituents selected from halo, (C₁₋₆) alkyl, fluoro(C₁₋₆) alkyl, OR⁷, SR⁷, NO₂, CN, COR⁷, CONR⁸R⁹, SO₂NR⁸R⁹, NR⁸SO₂R⁷, NR⁸R⁹, and optionally substituted phenyl; where R⁷ is hydrogen, (C₁₋₆) alkyl, fluorinated alkyl, or optionally substituted phenyl; and R⁸ and R⁹ are in each instance selected from hydrogen, (C₁₋₆) alkyl, or optionally substituted phenyl, including salts, solvates, and crystal forms thereof.

In one variation, the fused ring A is substituted with one or more substituents selected from (C₁₋₆) alkyl, halo, fluoro(C₁₋₆) alkyl, OR⁴, SR⁴, NO₂, CN, COR⁴, CONR⁵R⁶, SO₂NR⁵R⁶, NR⁵R⁶, NR⁵COR⁴, NR⁵SO²R⁴, or optionally substituted phenyl; where R⁴ is hydrogen, (C₁₋₆) alkyl, fluorinated (C₁₋₆) alkyl, or optionally substituted phenyl; and R⁵ and R⁶ are independently hydrogen, (C₁₋₆) alkyl, or optionally substituted phenyl; or R⁵ and R⁶ together with the attached nitrogen form an optionally substituted heterocyclyl; including salts, solvates, and crystal forms thereof.

Illustrative atypical antipsychotic compounds include, but are not limited to, clozapine, olanzapine and quetiapine

In one embodiment, a therapeutically effective amount of clozapine (also known as Clozaril, Leponex, Fazaclo, Froidir; Gen-Clozapine in Canada; Clozaril, Denzapine, Zaponex in the UK; Klozapol in Poland) is co-administered. As used herein, clozapine includes compounds of the formula. 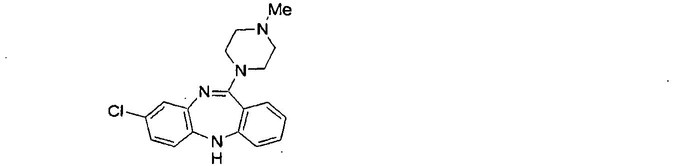 and pharmaceutically acceptable salts thereof.

In another embodiment of the methods described herein, a therapeutically effective amount of clozapine, or an analog or derivative thereof, or a pharmaceutically acceptable salt of the foregoing is co-administered to a patient in need of relief or suffering from multiple sclerosis. Additional analogs and derivatives that may be included in the methods described herein are described in US Patent No. 7,214,673.

Clozapine has been reported as an atypical antipsychotic drug because its profile of binding to serotonergic as well as dopamine receptors; its effects on various dopamine mediated behaviors also differ from those exhibited by more typical antipsychotic drugs. In particular, clozapine has been reported to interfere to a lower extent with the binding of dopamine at D1, D2, D3 and D5 receptors, and has a high affinity for the D4 receptor, but it does not induce catalepsy nor inhibit apomorphine-induced stereotypy in animal models as is seen with conventional neuroleptics. Without being bound by theory, this evidence may suggest that clozapine is preferentially more active at limbic than at striatal dopamine receptors and may explain the relative freedom of clozapine from extrapyramidal side effects together with strong anticholinergic activity

Clozapine has also been reported to be a partial agonist at the 5-HT1A receptor, putatively improving depression, anxiety, and negative/cognitive symptoms. Clozapine has also been reported to be a strong antagonist at different subtypes of adrenergic, cholinergic and histaminergic receptors, the last two being predominantly responsible for its side effect profile.

In one embodiment, 100 mg tablets available from Mylan Pharmaceuticals Inc. and 25 mg and 100 mg tablets (Clozaril) available from Novartis Pharmaceuticals are co-administered in the methods described herein. In another illustrative embodiment, clozapine, or an analog or derivative thereof, or a pharmaceutically acceptable salt of the foregoing, is co-administered at 100 mg b.i.d. In another embodiment, clozapine is co-administered in the range from about 12.5 to about 900 mg daily; or from about 150 to about 450 mg daily. Additional dosages for clozapine are described in US Patent No. 3,539,573.

In another embodiment of the methods described herein, clozapine treatment is commenced at a very low dose and increased slowly until a therapeutic dose is reached. Illustrative dosing protocols are described in Novartis Pharmaceuticals. "Clozaril Dosing Guide". Novartis Pharmaceuticals. Retrieved on 2007-06-29; (March 2008) "4.2.1 Antipsychotic drugs", British National Formulary, 55, p195. In severely ill and/or younger patients higher doses may be needed, while in the elderly much lower doses may be sufficient. Once the patient is stabilized and the maintenance dose has been determined, the greater part or all of the daily dose may be given at bedtime.

Norclozapine has been reported to be a primary metabolite of clozapine, which accumulates to, on average, 70% or so of the clozapine concentration in plasma at steady-state (trough sample i.e. pre-dose, ideally in the morning). Without being bound by theory, it is appreciated that the efficacy observable with clozapine in treating patients with multiple sclerosis may be due wholly or in part to norclozapine. It is understood that there may be substantial variation in the clozapine:norclozapine concentration ratio between individuals. It is believed herein that a steady-state plasma clozapine concentration of 0.35 to 0.6 mg/L should produce a clinical response in most patients.

In one embodiment not of the invention, a therapeutically effective amount of olanzapine (also known as Zyprexa, Zyprexa Zydis, Zalasta, Zolafren, Olzapin, or in combination with fluoxetine Symbyax) is co-administered. As used herein, olanzapine includes compounds of the formula. 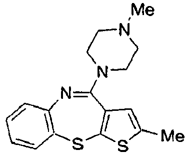 and pharmaceutically acceptable salts thereof.

In another embodiment not of the invention of the methods described herein, a therapeutically effective amount of olanzapine, or an analog or derivative thereof, or a pharmaceutically acceptable salt of the foregoing is co-administered to a patient in need of relief or suffering from multiple sclerosis. Illustrative analogs and derivatives of olanzapine that may be co-administered in the methods described herein include compounds of the formula 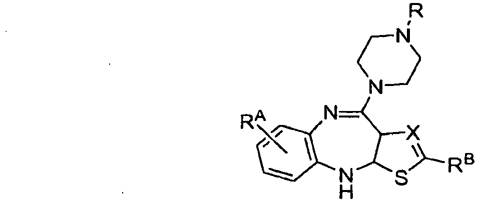 wherein R is hydrogen or (C₁₋₄) alkyl optionally substituted with OH, OR³, or OCH₂CH₂OH; where R³ is (C₁₋₂) alkyl;
R^{B} is hydrogen, (C₁₋₆) alkyl, halogen, fluorinated (C₁₋₆) alkyl, OR⁴, SR⁴, NO₂, CN, COR⁴, CONR⁵R⁶, SO₂NR⁵R⁶, NR⁵R⁶, NR⁵COR⁴, NR⁵SO₂R⁴, or optionally substituted phenyl; where R⁴ is hydrogen, (C₁₋₆) alkyl, fluorinated (C₁₋₆) alkyl, or optionally substituted phenyl; and R⁵ and R⁶ are independently hydrogen, (C₁₋₆) alkyl, or optionally substituted phenyl; or R⁵ and R⁶ together with the attached nitrogen form an optionally substituted heterocyclyl;
R^{A} is hydrogen or one or two substituents selected from halogen, (C₁₋₆) alkyl, fluorinated (C₁₋₆) alkyl, OR⁷, SR⁷, NO₂, CN, COR⁷, CONR⁸R⁹, SO₂NR⁸R⁹, NR⁸SO₂R⁷, NR⁸R⁹, and optionally substituted phenyl; where R⁷ is hydrogen, (C₁₋₆) alkyl, fluorinated alkyl, or optionally substituted phenyl; and R⁸ and R⁹ are in each instance selected from hydrogen, (C₁₋₆) alkyl, or optionally substituted phenyl; and X is CH or N; including salts, solvates, and crystal forms thereof. Additional analogs and derivatives that may be included in the methods described herein are described in US Patent No. 7,214,673.

In another embodiment, olanzapine or a thienobenzodiazepines analog thereof is co-administered in the methods described herein.

It is appreciated that like most atypical antipsychotics, compared to the older typical ones, olanzapine has a lower affinity for histamine, cholinergic muscarinic and alpha adrenergic receptors. The mode of action of olanzapine's antipsychotic activity is unknown. It is understood that antagonism of dopamine receptors is associated with extrapyramidal effects such as tardive dyskinesia. In addition, it is understood that antagonizing H1 histamine receptors causes sedation and may cause weight gain, although antagonistic actions at 5-HT2C receptors have also been implicated in weight gain. Olanzapine is structurally similar to clozapine, and is classified as a thienobenzodiazepine. It has been reported that olanzapine has a higher affinity for 5-HT2 serotonin receptors than D2 dopamine receptors. Without being bound by theory, it is appreciated herein that such higher affinity may account for the efficacy observable in treating patients suffering from multiple sclerosis.

Olanzapine is available from Eli Lilly as Symbyax capsules, Zyprexa tablets, Zyprexa intramuscular injection, and Zyprexa ZYDIS orally disintegrating tablets. Olanzapine is available as a tablet in strengths of 2.5 mg, 5 mg, 7.5 mg, 10 mg, 15 mg and 20 mg and orally disintegrating wafers (known as Zydis), which dissolve on the tongue, in strengths of 5 mg, 10 mg, 15 mg and 20 mg. It is also available as a rapid-acting intramuscular injection for short-term acute use. In one illustrative embodiment, olanzapine is co-administered orally once daily at 3 mg, 6 mg, or 12 mg, alone or in combination with fluoxetine. In another illustrative embodiment, olanzapine, for example Zyprexa ZYDIS orally disintegrating tablets, is co-administered once daily at 5 mg, 10, mg, 15 mg, or 20 mg. In another illustrative embodiment, olanzapine, for example Zyprexa intramuscular injection, is co-administered once daily at 10 mg. In another embodiment, olanzapine is co-administered in the range from about 0.25 to about 100 mg, once/day; or from about 1 to about 30 mg, once/day; or about 1 to about 25 mg once/day. Additional dosages for olanzapine are described in US Patent No. 5,229,382.

It is understood that the dose may be adjusted depending on the patient response to the drug, or depending on certain medical problems the patient may have. In one embodiment, the therapeutically effective amount is co-administered once daily before bed as it may highly sedating.

In one embodiment, a therapeutically effective amount of quetiapine (also known as Seroquel or Seroquel XR extended release (AstraZeneca) and Ketipinor (Orion Pharma)) is co-administered. As used herein, quetiapine includes compounds of the formula. 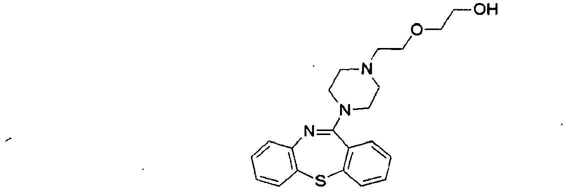 and pharmaceutically acceptable salts thereof, such as fumarate salts.

In another embodiment of the methods described herein, a therapeutically effective amount of quetiapine, or an analog or derivative thereof, or a pharmaceutically acceptable salt of the foregoing is co-administered to a patient in need of relief or suffering from multiple sclerosis. Illustrative analogs and derivatives of quetiapine that may be co-administered in the methods described herein include compounds of the formula 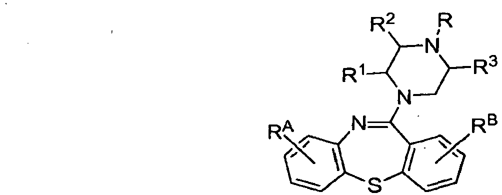 wherein R^{A} and R^{B} each represent hydrogen or one or more substituents each independently selected from hydroxyl, halo, C₁₋₄ alkyl, amino, nitro, cyano, C₁₋₄ haloalkyl, C₁₋₄ alkoxy, C₁₋₄ haloalkoxy, C₁₋₄ alkoxycarbonyl, C₁₋₄ CO--, cyclo-C₃₋₇ alkyl, C₁₋₄ thiolalkyl, C₁₋₄ alkylthio, halo-substituted C₁₋₄ alkylthio, cyanothio, tetrazolyl, N-piperidinyl, N-piperazinyl, N-morpholinyl, acetamido, C₁₋₄ alkylsulfonyl, halosulfonyl, halo-substituted C₁₋₄ alkylsulfonyl, C₁₋₄ alkylsulfoxyl SO₂ NH₂, C₁₋₄,alkylseleno, and OSO₃H;
R, R¹, and R² are each independently selected from H and C₁₋₆ alkyl optionally substituted with a substituent selected from hydroxyl, halo, C₁₋₄ alkyl or C₁₋₄ alkoxy;
R³ is C₁₋₆ alkyl optionally substituted with a substituent selected from hydroxyl, halo, C₁₋₄ alkyl or C₁₋₄ alkoxy; including salts, solvates, and crystal forms thereof. Additional analogs and derivatives that may be included in the methods described herein are described in US Patent No. 5,824,676.

It has been reported that the antipsychotic effect of quetiapine is mediated through antagonist activity at dopamine and serotonin receptors. Specifically the D1 and D2 dopamine receptor, the alpha-1 and alpha-2 adrenergic receptor, and 5-HT1A and 5-HT2 serotonin receptor subtypes are believed herein to be antagonized. It has been reported that serial PET scans evaluating the D2 receptor occupancy of quetiapine demonstrate that quetiapine very rapidly disassociates from the D2 receptor. Quetiapine has also been reported to have an antagonistic effect on the histamine H1 receptor.

Quetiapine is available under the brand name Seroquel. It is available in 25 mg, 50 mg, 100 mg, 200 mg, 300 mg, and 400 mg tablets. In one embodiment, compositions including quetiapine, and analogs and derivatives thereof, and pharmaceutically acceptable salts thereof, are included in a unit dose form such as a tablet, capsule or ampoule. Suitable unit doses i.e. therapeutically effective amounts; can be determined during clinical trials designed appropriately for each of the conditions for which administration of a chosen compound is indicated and may vary depending on the desired clinical endpoint. It is understood that dosage sizes appropriate for administering the compounds of the examples may be in the range from about 0.1 to about 500 mg/kg body weight eg. 0.1 to about 100 mg/kg body weight, and may be co-administered in a frequency appropriate for initial and maintenance treatments. In another embodiment, quetiapine is co-administered in the range from about 1.0 to about 40 mg/kg given once daily or in divided doses. Additional dosages for quetiapine are described in US Patent No. 4,879,288. In another illustrative embodiment, quetiapine is co-administered as its (E)-2-butenedioate (2:1) salt.

In another illustrative embodiment, Seroquel extended release is administered once daily initially at 300 mg, and subsequently increasing to 400 mg to 800 mg. In another embodiment, Seroquel is co-administered at a dose of less than 100 mg daily, in a single or divided dose, and is increased to a dose of between 400 mg and 800 mg daily, in a single or divided dose. It is understood that the divided dose in each of the foregoing may be co-administered qd, bid, or tid.

In one embodiment of each of the foregoing tricyclic compounds, the compounds are co-administered for example to adult humans, in dosages in the range from 0.1 to 500 mg, in the range from 0.25 mg to 100 mg, in the range from 0.25 mg to 50 mg per day. In another embodiment, a once a day dosage is sufficient, although divided doses may be co-administered.

In another embodiment, compositions and methods are described herein that also include one or more immunosuppressive drugs. Illustrative immunosuppressive drugs include, but are not limited to, azathioprine, mycophenolate mofetil, corticosteroids, mitoxantrone, cyclophophosphamide, methotrexate, cyclosporine, and the like, and pharmaceutically acceptable salts thereof. It is appreciated that some immunosuppressive drugs are monoclonal antibodies. In one variation, illustrative immunosuppressive drugs are monoclonal antibodies. Illustrative immunosuppressive drugs include, but are not limited to, natalizumab, daclizumab, alemtuzumab, rituximab, and the like. In another variation, the immunosuppressive drug is azathioprine, or pharmaceutically acceptable salts thereof.

In another embodiment, compositions and methods are described herein that also include one or more immunomodulatory drugs. Illustrative immunomodulatory drugs include, but are not limited to, interferon beta-lb, interferon beta-la, glatiramer acetate (copaxone), natalizumab, rituximab, daclizumab, BG12, fingolimod, laquinimod, and the like, and pharmaceutically acceptable salts thereof. It is appreciated that some immunomodulatory drugs are monoclonal antibodies. In one variation, illustrative immunomodulatory drugs are monoclonal antibodies. Illustrative immunomodulatory drugs include, but are not limited to, natalizumab, daclizumab, alemtuzumab and rituximab.

It is to be understood that in any of the method or composition embodiments described herein, the corresponding acid addition salt of any compound may be administered in addition to or instead of the corresponding neutral compound. Illustrative acid salts may be formed from, but at not limited to, inorganic acids such as hydrohalic acids, including hydrochloric or hydrobromic acid; sulfuric; nitric; phosphoric, and the like acids; and organic acids such as acetic, propanoic, hydroxyacetic, lactic, pyruvic, oxalic, malonic, succinic, maleic, fumaric, malic, tartaric, citric, methanesulfonic, ethanesulfonic, benzenesulfonic, p-toluenesulfonic, cyclamic, salicylic, p-aminosalicylic, pamoic and the like acids. In addition, it is to be understood that bis salts may be formed and administered, such as the dihydrochloride acid salt.

As used herein, the term "alkyl" includes a chain of carbon atoms, which is optionally branched. It is to be understood that alkyl is advantageously of limited length, including C₁-C₂₄, C₁-C₁₂, C₁-C₈, C₁-C₆, and C₁-C₄. It is appreciated herein that shorter alkyl groups add less lipophilicity to the compound and accordingly will have different pharmacokinetic behavior. As used herein, the term "cycloalkyl" includes a chain of carbon atoms, which is optionally branched, and where at least a portion of the chain in cyclic. It is to be understood that chain forming cycloalkyl is advantageously of limited length, including C₃-C₂₄, C₃-C₁₂, C₃-C₈, C₃-C₆, and C₃-C₄. It is appreciated herein that shorter alkyl groups add less lipophilicity to the compound and accordingly will have different pharmacokinetic behavior.

As used herein, the term "heteroalkyl" includes a chain of atoms that includes both carbon and at least one heteroatom, and is optionally branched. Illustrative heteroatoms include nitrogen, oxygen, and sulfur. In certain variations, illustrative heteroatoms also include phosphorus, and selenium. As used herein, the term "heterocyclyl" including heterocycle includes a chain of atoms that includes both carbon and at least one heteroatom, and is optionally branched, where at least a portion of the chain is cyclic. Illustrative heteroatoms include nitrogen, oxygen, and sulfur. In certain variations, illustrative heteroatoms also include phosphorus, and selenium. Illustrative heteocycles include, but are not limited to, tetrahydrofuryl, pyrrolidinyl, tetrahydropyranyl, piperidinyl, morpholinyl; piperazinyl, homopiperazinyl and quinuclidinyl.

As used herein, the term "aryl" includes monocyclic and polycyclic aromatic carbocyclic and aromatic heterocyclic groups, each of which may be optionally substituted. As used herein, the term "heteroaryl" includes aromatic heterocyclic groups, each of which may be optionally substituted. Illustrative carbocyclic aromatic groups described herein include, but are not limited to, phenyl and naphthyl. Illustrative heterocyclic aromatic groups include, but are not limited to, pyridinyl, pyrimidinyl, pyrazinyl, triazinyl, tetrazinyl, quinolinyl, quinazolinyl, quinoxalinyl, thienyl, pyrazolyl, imidazolyl, oxazolyl, thiazolyl, isoxazolyl, isothiazolyl, oxadiazolyl, thiadiazolyl, triazolyl, benzimidazolyl, benzoxazolyl, benzthiazolyl, benzisoxazolyl and benzisothiazolyl.

As used herein, the term "amino" includes the group NH₂, alkylamino, and dialkylamino, where the two alkyl groups in dialkylamino may be the same or different, i.e. alkylalkylamino. Illustratively, amino includes methylamino, ethylamino, dimethylamino and methylethylamino. In addition, it is to be understood that when amino modifies or is modified by another term, such as aminoalkyl, or acylamino, the above variations of the term amino are included therein. Illustratively, aminoalkyl includes H₂N-alkyl, methylaminoalkyl, ethylaminoalkyl, dimethylaminoalkyl and methylethylaminoalkyl. Illustratively, acylamino includes acylmethylamino and acylethylamino.

As used herein, the term "optionally substituted amino" includes derivatives of amino as described herein, such as, but not limited to, acylamino, urea, and carbamate.

The term "optionally substituted" as used herein includes the replacement of hydrogen atoms with other functional groups on the radical that is optionally substituted. Such other functional groups illustratively include, but are not limited to, amino, hydroxyl, halo, thiol, alkyl, haloalkyl, heteroalkyl, aryl, arylalkyl, arylheteroalkyl, nitro, sulfonic acids and derivatives thereof, carboxylic acids and derivatives thereof.

The term "optionally substituted aryl" as used herein includes the replacement of hydrogen atoms with other functional groups on the aryl that is optionally substituted. Such other functional groups illustratively include, but are not limited to, amino, hydroxyl, halo, thiol, alkyl, haloalkyl, heteroalkyl, aryl, arylalkyl, arylheteroalkyl, nitro, sulfonic acids and derivatives thereof, carboxylic acids and derivatives thereof.

Illustrative substituents include, but are not limited to, a radical -(CH₂)ₘZ, where m is an integer from 0-6 and Z is selected from halogen, hydroxy, alkanoyloxy, including C₁-C₆ alkanoyloxy, optionally substituted aroyloxy, alkyl, including C₁-C₆ alkyl, alkoxy, including C₁-C₆ alkoxy, cycloalkyl, including C₃-C₈ cycloalkyl, cycloalkoxy, including C₃-C₈ cycloalkoxy, alkenyl, including C₂-C₆ alkenyl, alkynyl, including C₂-C₆ alkynyl, haloalkyl, including C₁-C₆ haloalkyl, haloalkoxy, including C₁-C₆ haloalkoxy, halocycloalkyl, including C₃-C₈ halocycloalkyl, halocycloalkoxy, including C₃-C₈ halocycloalkoxy, amino, C₁-C₆ alkylamino, (C₁-C₆ alkyl)(C₁-C₆ alkyl)amino, alkylcarbonylamino, N-(C₁-C₆ alkyl)alkylcarbonylamino, aminoalkyl, C₁-C₆ alkylaminoalkyl, (C₁-C₆ alkyl)(C₁-C₆ alkyl)aminoalkyl, alkylcarbonylaminoalkyl, N-(C₁-C₆ alkyl)alkylcarbonylaminoalkyl, cyano, and nitro; or Z is selected from -CO₂R⁴ and -CONR⁵R⁶, where R⁴, R⁵, and R⁶ are each independently selected in each occurrence from hydrogen, C₁-C₆ alkyl, and aryl-C₁-C₆ alkyl.

The term "prodrug" as used herein generally refers to any compound that when administered to a biological system generates a biologically active compound as a result of one or more spontaneous chemical reaction(s), enzyme-catalyzed chemical reaction(s), and/or metabolic chemical reaction(s), or a combination thereof. In vivo, the prodrug is typically acted upon by an enzyme (such as esterases, amidases, phosphatases, and the like), simple biological chemistry, or other process in vivo to liberate or regenerate the more pharmacologically active drug. This activation may occur through the action of an endogenous host enzyme or a non-endogenous enzyme that is administered to the host preceding, following, or during administration of the prodrug. Additional details of prodrug use are described in U.S. Pat. No. 5,627,165; and Pathalk et al., Enzymic protecting group techniques in organic synthesis, Stereosel. Biocatal. 775-797 (2000). It is appreciated that the prodrug is advantageously converted to the original drug as soon as the goal, such as targeted delivery, safety, stability, and the like is achieved, followed by the subsequent rapid elimination of the released remains of the group forming the prodrug.

Prodrugs may be prepared from the compounds described herein by attaching groups that ultimately cleave in vivo to one or more functional groups present on the compound, such as -OH-, -SH, -CO₂H, -NR₂. Illustrative prodrugs include but are not limited to carboxylate esters where the group is alkyl, aryl, aralkyl, acyloxyalkyl, alkoxycarbonyloxyalkyl as well as esters of hydroxyl, thiol and amines where the group attached is an acyl group, an alkoxycarbonyl, aminocarbonyl, phosphate or sulfate. Illustrative esters, also referred to as active esters, include but are not limited to 1-indanyl, N-oxysuccinimide; acyloxyalkyl groups such as acetoxymethyl, pivaloyloxymethyl, (β-acetoxyethyl, (β-pivaloyloxyethyl, 1-(cyclohexylcarbonyloxy)prop-1-yl, (1 -arninoethyl)carbonyloxymethyl, and the like; alkoxycarbonyloxyalkyl groups, such as ethoxycarbonyloxymethyl, α-ethoxycarbonyloxyethyl and β-ethoxycarbonyloxyethyl; dialkylaminoalkyl groups, including di-lower alkylamino alkyl groups, such as dimethylaminomethyl, dimethylaminoethyl, diethylaminomethyl, diethylaminoethyl, and the like; 2-(alkoxycarbonyl)-2-alkenyl groups such as 2-(isobutoxycarbonyl) pent-2-enyl, 2-(ethoxycarbonyl)but-2-enyl, and the like; and lactone groups such as phthalidyl and dimethoxyphthalidyl.

Further illustrative prodrugs contain a chemical moiety, such as an amide or phosphorus group functioning to increase solubility and/or stability of the compounds described herein. Further illustrative prodrugs for amino groups include, but are not limited to, (C₃-C₂₀)alkanoyl; halo-(C₃-C₂₀)alkanoyl; (C₃-C₂₀)alkenoyl; (C₄-C₇)cycloalkanoyl; (C₃-C₆)-cycloalkyl(C₂-C₁₆)alkanoyl; optionally substituted aroyl, such as unsubstituted aroyl or aroyl substituted by 1 to 3 substituents selected from the group consisting of halogen, cyano, trifluoromethanesulphonyloxy, (C₁-C₃)alkyl and (C₁-C₃)alkoxy, each of which is optionally further substituted with one or more of 1 to 3 halogen atoms; optionally substituted aryl(C₂-C₆)alkanoyl, such as the aryl radical being unsubstituted or substituted by 1 to 3 substituents selected from the group consisting of halogen, (C₁-C₃)alkyl and (C₁-C₃)alkoxy, each of which is optionally further substituted with 1 to 3 halogen atoms; and optionally substituted heteroarylalkanoyl having one to three heteroatoms selected from O, S and N in the heteroaryl moiety and 2 to 10 carbon atoms in the alkanoyl moiety, such as the heteroaryl radical being unsubstituted or substituted by 1 to 3 substituents selected from the group consisting of halogen, cyano, trifluoromethanesulphonyloxy, (C₁-C₃)alkyl, and (C₁-C₃)alkoxy, each of which is optionally further substituted with 1 to 3 halogen atoms. The groups illustrated are exemplary, not exhaustive, and may be prepared by conventional processes.

It is understood that the prodrugs themselves may not possess significant biological activity, but instead undergo one or more spontaneous chemical reaction(s), enzyme-catalyzed chemical reaction(s), and/or metabolic chemical reaction(s), or a combination thereof after administration in vivo to produce the compound described herein that is biologically active or is a precursor of the biologically active compound. However, it is appreciated that in some cases, the prodrug is biologically active. It is also appreciated that prodrugs may often serves to improve drug efficacy or safety through improved oral bioavailability, pharmacodynamic half-life, and the like. Prodrugs also refer to derivatives of the compounds described herein that include groups that simply mask undesirable drug properties or improve drug delivery. For example, one or more compounds described herein may exhibit an undesirable property that is advantageously blocked or minimized may become pharmacological, pharmaceutical, or pharmacokinetic barriers in clinical drug application, such as low oral drug absorption, lack of site specificity, chemical instability, toxicity, and poor patient acceptance (bad taste, odor, pain at injection site, and the like), and others. It is appreciated herein that a prodrug, or other strategy using reversible derivatives, can be useful in the optimization of the clinical application of a drug.

The term "therapeutically effective amount" as used herein, refers to that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated. In one aspect, the therapeutically effective amount is that which may treat or alleviate the disease or symptoms of the disease at a reasonable benefit/risk ratio applicable to any medical treatment. However, it is to be understood that the total daily usage of the compounds and compositions described herein may be decided by the attending physician within the scope of sound medical judgment. The specific therapeutically-effective dose level for any particular patient will depend upon a variety of factors, including the disorder being treated and the severity of the disorder; activity of the specific compound employed; the specific composition employed; the age, body weight, general health, gender and diet of the patient: the time of administration, route of administration, and rate of excretion of the specific compound employed; the duration of the treatment; drugs used in combination or coincidentally with the specific compound employed; and like factors well known to the researcher, veterinarian, medical doctor or other clinician of ordinary skill.

In addition, in those embodiments described herein drawn to combination therapy comprising administration of compounds according to the first aspect of the invention, and/or one or more dimebolins, and/or one or more NMDA antagonists and/or one or more statin, "therapeutically effective amount" refers to that amount of the combination of agents taken together so that the combined effect elicits the desired biological or medicinal response. For example, the therapeutically effective amount of pirlindole and dimebon, pirlindole and simvastatin, and the like, would be the amount of pirlindole and the amount dimebon, or the amount of pirlindole and the amount of the simvastatin, and the like that when taken together or sequentially have a combined effect that is therapeutically effective. Further, it is appreciated that in some embodiments of such methods that include co-administration, the amount of tetracyclic pyrazinoindoles, dimebon, and/or simvastatin, and the like when taken individually may or may not be therapeutically effective.

It is also appreciated that the therapeutically effective amount, whether referring to monotherapy or combination therapy, is advantageously selected with reference to any toxicity, or other undesirable side effect, that might occur during administration of one or more of the compounds described herein. Further, it is appreciated that the co-therapies described herein may allow for the administration of lower doses of compounds that show such toxicity, or other undesirable side effect, where those lower doses are below thresholds of toxicity or lower in the therapeutic window than would otherwise be administered in the absence of a cotherapy.

As used herein, the term "composition" generally refers to any product comprising the specified ingredients in the specified amounts, as well as any product which results, directly or indirectly, from combinations of the specified ingredients in the specified amounts. It is to be understood that the compositions described herein may be prepared from isolated compounds described herein or from salts, solutions, hydrates, solvates, and other forms of the compounds described herein. It is also to be understood that the compositions may be prepared from various amorphous, non-amorphous, partially crystalline, crystalline, and/or other morphological forms of the compounds described herein. It is also to be understood that the compositions maybe prepared from various hydrates and/or solvates of the compounds described herein. Accordingly, such pharmaceutical compositions that recite compounds described herein are to be understood to include each of, or any combination of, the various morphological forms and/or solvate or hydrate forms of the compounds described herein. Illustratively, compositions may include one or more carriers, diluents, and/or excipients. The compounds described herein, or compositions containing them, may be formulated in a therapeutically effective amount in any conventional dosage forms appropriate for the methods described herein, and include one or more carriers, diluents, and/or excipients therefor. Such formulation compositions may be administered by a wide variety of conventional routes for the methods described herein, and in a wide variety of dosage formats, utilizing known procedures. See generally, Remington: The Science and Practice of Pharmacy, (21st ed., 2005).

The term "administering" as used herein includes all means of introducing the compounds and compositions described herein to the patient, including, but are not limited to, oral (po), intravenous (iv), intramuscular (im), subcutaneous (sc), transdermal, inhalation, buccal, ocular, sublingual, and rectal in dosage form unit formulations containing conventional nontoxic pharmaceutically-acceptable carriers, adjuvants and vehicles. Compounds may also be administered directly to the nervous system including, but not limited to, intracerebral, intraventricular, intracerebroventricular, intrathecal, intracisternal, intraspinal and/or peri-spinal routes of administration by delivery via intracranial or intravertebral needles and/or catheters with or without pump devices.

It is to be understood that in the methods described herein do not form part of the present invention, the individual components of a coadministration, or combination can be administered by any suitable means, contemporaneously, simultaneously, sequentially, separately or in a single pharmaceutical formulation. Where the co-administered compounds or compositions are administered in separate dosage forms, the number of dosages administered per day for each compound may be the same or different. The compounds or compositions may be administered via the same or different routes of administration. The compounds or compositions may be administered according to simultaneous or alternating regimens, at the same or different times during the course of the therapy, concurrently in divided or single forms.

In addition to the foregoing illustrative dosages and dosing protocols, it is to be understood that an effective amount of any one or a mixture of the compounds described herein can be readily determined by the attending diagnostician or physician by the use of known techniques and/or by observing results obtained under analogous circumstances. In determining the effective amount or dose, a number of factors are considered by the attending diagnostician or physician, including, but not limited to the species of mammal, including human, its size, age, and general health, the specific disease or disorder involved, the degree of or involvement or the severity of the disease or disorder, the response of the individual patient, the particular compound administered, the mode of administration, the bioavailability characteristics of the preparation administered, the dose regimen selected, the use of concomitant medication, and other relevant circumstances.

In making the pharmaceutical compositions of the compounds described herein, a therapeutically effective amount of one or more compounds in any of the various forms described herein may be mixed with one or more excipients, diluted by one or more excipients, or enclosed within such a carrier which can be in the form of a capsule, sachet, paper, or other container. Excipients may serve as a diluent, and can be solid, semi-solid, or liquid materials, which act as a vehicle, carrier or medium for the active ingredient. Thus, the formulation compositions can be in the form of tablets, pills, powders, lozenges, sachets, cachets, elixirs, suspensions, emulsions, solutions, syrups, aerosols (as a solid or in a liquid medium), ointments, soft and hard gelatin capsules, suppositories, sterile injectable solutions, and sterile packaged powders. The compositions may contain anywhere from about 0.1% to about 99.9% active ingredients, depending upon the selected dose and dosage form. Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. The formulations can additionally include lubricating agents such as talc, magnesium stearate, and mineral oil; wetting agents; emulsifying and suspending agents; preserving agents such as methyl- and propylhydroxybenzoates; sweetening agents; and flavoring agents. The compositions can be formulated so as to provide quick, sustained or delayed release of the active ingredient after administration to the patient by employing procedures known in the art. It is appreciated that the carriers, diluents, and excipients used to prepare the compositions described herein are advantageously GRAS (Generally Regarded as Safe) compounds.

Examples of emulsifying agents are naturally occurring gums (e.g., gum acacia or gum tragacanth) and naturally occurring phosphatides (e.g., soybean lecithin and sorbitan monooleate derivatives). Examples of antioxidants are butylated hydroxy anisole (BHA), ascorbic acid and derivatives thereof, tocopherol and derivatives thereof, butylated hydroxy anisole, and cysteine. Examples of preservatives are parabens, such as methyl or propyl p-hydroxybenzoate, and benzalkonium chloride. Examples of humectants are glycerin, propylene glycol, sorbitol, and urea. Examples of penetration enhancers are propylene glycol, DMSO, triethanolamine, N,N-dimethylacetamide, N,N-dimethylformamide, 2-pyrrolidone and derivatives thereof, tetrahydrofurfuryl alcohol, and AZONE. Examples of chelating agents are sodium EDTA, citric acid, and phosphoric acid. Examples of gel forming agents are CARBOPOL, cellulose derivatives, bentonite, alginates, gelatin and polyvinylpyrrolidone. Examples of ointment bases are beeswax, paraffin, cetyl palmitate, vegetable oils, sorbitan esters of fatty acids (Span), polyethylene glycols, and condensation products between sorbitan esters of fatty acids and ethylene oxide (e.g., polyoxyethylene sorbitan monooleate (TWEEN)).

Solid Dosage Forms for Oral Use. Formulations for oral use include tablets containing the active ingredient(s) in a mixture with non-toxic pharmaceutically acceptable excipients. These excipients may be, for example, inert diluents or fillers (e.g., sucrose, sorbitol, sugar, mannitol, microcrystalline cellulose, starches including potato starch, calcium carbonate, sodium chloride, lactose, calcium phosphate, calcium sulfate, or sodium phosphate); granulating and disintegrating agents (e.g., cellulose derivatives including microcrystalline cellulose, starches including potato starch, croscarmellose sodium, alginates, or alginic acid); binding agents (e.g., sucrose, glucose, sorbitol, acacia, alginic acid, sodium alginate, gelatin, starch, pregelatinized starch, microcrystalline cellulose, magnesium aluminum silicate, carboxymethylcellulose sodium, methylcellulose, hydroxypropyl methylcellulose, ethylcellulose, polyvinylpyrrolidone, or polyethylene glycol); and lubricating agents, glidants, and antiadhesives (e.g., magnesium stearate, zinc stearate, stearic acid, silicas, hydrogenated vegetable oils, or talc). Other pharmaceutically acceptable excipients can be colorants, flavoring agents, plasticizers, humectants and buffering agents.

The tablets may be uncoated or they may be coated by known techniques, optionally to delay disintegration and absorption in the gastrointestinal tract and thereby providing a sustained action over a longer period. The coating may be adapted to release the active drug substance in a predetermined pattern (e.g., in order to achieve a controlled release formulation) or it may be adapted not to release the active drug substance until after passage of the stomach (enteric coating). The coating may be a sugar coating, a film coating (e.g., based on hydroxypropyl methylcellulose, methylcellulose, methyl hydroxyethylcellulose, hydroxypropylcellulose, carboxymethylcellulose, acrylate copolymers, polyethylene glycols and/or polyvinylpyrrolidone), or an enteric coating (e.g., based on methacrylic acid copolymer, cellulose acetate phthalate, hydroxypropyl methylcellulose phthalate, hydroxypropyl methylcellulose acetate succinate, polyvinyl acetate phthalate, shellac, and/or ethylcellulose). Furthermore, a time delay material such as, e.g., glyceryl monostearate or glyceryl distearate may be employed.

The solid tablet compositions may include a coating adapted to protect the composition from unwanted chemical changes, (e.g., chemical degradation prior to the release of the active drug substance). The coating may be applied on the solid dosage form in a similar manner as that described in Encyclopedia of Pharmaceutical Technology.

Controlled Release Oral Dosage Forms. Controlled release compositions for oral use may, e.g., be constructed to release the active drug by controlling the dissolution and/or the diffusion of the active drug substance. Illustrative sustained release formulations are described in US Patent Nos. 3,847,770; 3,916,899; 3,536,809; 3,598,123; 3,630,200; 4,008,719; 4,687,610; 4,769,027; 5,674,533; 5,059,595; 5,591,767; 5,120,548; 5,073,543; 5,639,476; 5,354,566; and 5,733,566, the disclosures of which are incorporated herein by reference.

Dissolution or diffusion controlled release can be achieved by appropriate coating of a tablet, capsule, pellet, or granulate formulation of compounds, or by incorporating the compound into an appropriate matrix. A controlled release coating may include one or more of the coating substances mentioned above and/or, e.g., shellac, beeswax, glycowax, castor wax, carnauba wax, stearyl alcohol, glyceryl monostearate, glyceryl distearate, glycerol palmitostearate, ethylcellulose, acrylic resins, dl-polylactic acid, cellulose acetate butyrate, polyvinyl chloride, polyvinyl acetate, vinyl pyrrolidone, polyethylene, polymethacrylate, methylmethacrylate, 2-hydroxymethacrylate, methacrylate hydrogels, 1,3 butylene glycol, ethylene glycol methacrylate, and/or polyethylene glycols. In a controlled release matrix formulation, the matrix material may also include, e.g., hydrated metylcellulose, carnauba wax and stearyl alcohol, carbopol 934, silicone, glyceryl tristearate, methyl acrylate-methyl methacrylate, polyvinyl chloride, polyethylene, and/or halogenated fluorocarbon.

A controlled release composition containing one or more of the compounds of the claimed combinations may also be in the form of a buoyant tablet or capsule (i.e., a tablet or capsule that, upon oral administration, floats on top of the gastric content for a certain period of time). A buoyant tablet formulation of the compound(s) can be prepared by granulating a mixture of the drug(s) with excipients and 20-75% w/w of hydrocolloids, such as hydroxyethylcellulose, hydroxypropylcellulose, or hydroxypropylmethylcellulose. The obtained granules can then be compressed into tablets. On contact with the gastric juice, the tablet forms a substantially water-impermeable gel barrier around its surface. This gel barrier takes part in maintaining a density of less than one, thereby allowing the tablet to remain buoyant in the gastric juice.

Liquids for Oral Administration. Powders, dispersible powders, or granules suitable for preparation of an aqueous suspension by addition of water are convenient dosage forms for oral administration. Formulation as a suspension provides the active ingredient in a mixture with a dispersing or wetting agent, suspending agent, and one or more preservatives. Suitable dispersing or wetting agents are, for example, naturally-occurring phosphatides (e.g., lecithin or condensation products of ethylene oxide with a fatty acid, a long chain aliphatic alcohol, or a partial ester derived from fatty acids) and a hexitol or a hexitol anhydride (e.g., polyoxyethylene stearate, polyoxyethylene sorbitol monooleate, polyoxyethylene sorbitan monooleate, and the like). Suitable suspending agents are, for example, sodium carboxymethylcellulose, methylcellulose and sodium alginate.

Parenteral Compositions. The pharmaceutical composition may also be administered parenterally by injection, infusion or implantation (intravenous, intramuscular, subcutaneous, or the like) in dosage forms, formulations, or via suitable delivery devices or implants containing conventional, non-toxic pharmaceutically acceptable carriers and adjuvants. The formulation and preparation of such compositions are well known to those skilled in the art of pharmaceutical formulation. Formulations can be found in Remington: The Science and Practice of Pharmacy.

Compositions for parenteral use may be provided in unit dosage forms (e.g., in single-dose ampoules), or in vials containing several doses and in which a suitable preservative may be added (see below). The composition may be in form of a solution, a suspension, an emulsion, an infusion device, or a delivery device for implantation, or it may be presented as a dry powder to be reconstituted with water or another suitable vehicle before use. Apart from the active drug(s), the composition may include suitable parenterally acceptable carriers and/or excipients. The active drug(s) may be incorporated into microspheres, microcapsules, nanoparticles, liposomes, or the like for controlled release. Furthermore, the composition may include suspending, solubilizing, stabilizing, pH-adjusting agents, and/or dispersing agents.

As indicated above, the pharmaceutical compositions described herein may be in the form suitable for sterile injection. To prepare such a composition, the suitable active drug(s) are dissolved or suspended in a parenterally acceptable liquid vehicle. Among acceptable vehicles and solvents that may be employed are water, water adjusted to a suitable pH by addition of an appropriate amount of hydrochloric acid, sodium hydroxide or a suitable buffer, 1,3-butanediol, Ringer's solution, and isotonic sodium chloride solution. The aqueous formulation may also contain one or more preservatives (e.g., methyl, ethyl or n-propyl p-hydroxybenzoate). In cases where one of the compounds is only sparingly or slightly soluble in water, a dissolution enhancing or solubilizing agent can be added, or the solvent may include 10-60% w/w of propylene glycol.

Controlled Release Parenteral Compositions. Controlled release parenteral compositions may be in form of aqueous suspensions, microspheres, microcapsules, magnetic microspheres, oil solutions, oil suspensions, or emulsions. Alternatively, the active drug(s) may be incorporated in biocompatible carriers, liposomes, nanoparticles, implants, or infusion devices. Materials for use in the preparation of microspheres and/or microcapsules are, e.g., biodegradable/bioerodible polymers such as polygalactin, poly-(isobutyl cyanoacrylate), poly(2-hydroxyethyl-L-glutamnine) and, poly(lactic acid). Biocompatible carriers that may be used when formulating a controlled release parenteral formulation are carbohydrates (e.g., dextrans), proteins (e.g., albumin), lipoproteins, or antibodies. Materials for use in implants can be non-biodegradable (e.g., polydimethyl siloxane) or biodegradable (e.g., poly(caprolactone), poly(lactic acid), poly(glycolic acid) or poly(ortho esters)).

Rectal Compositions. For rectal application, suitable dosage forms for a composition include suppositories (emulsion or suspension type), and rectal gelatin capsules (solutions or suspensions). In a typical suppository formulation, the active drug(s) are combined with an appropriate pharmaceutically acceptable suppository base such as cocoa butter, esterified fatty acids, glycerinated gelatin, and various water-soluble or dispersible bases like polyethylene glycols and polvoxyethylene sorbitan fatty acid esters. Various additives, enhancers, or surfactants may be incorporated.

Compositions for Inhalation. For administration by inhalation, typical dosage forms include nasal sprays and aerosols. In a typically nasal formulation, the active ingredient(s) are dissolved or dispersed in a suitable vehicle. The pharmaceutically acceptable vehicles and excipients (as well as other pharmaceutically acceptable materials present in the composition such as diluents, enhancers, flavoring agents, and preservatives) are selected in accordance with conventional pharmaceutical practice in a manner understood by the persons skilled in the art of formulating pharmaceuticals.

Percutaneous and Topical Compositions. The pharmaceutical compositions may also be administered topically on the skin for percutaneous absorption in dosage forms or formulations containing conventionally non-toxic pharmaceutical acceptable carriers and excipients including microspheres and liposomes. The formulations include creams, ointments, lotions, liniments, gels, hydrogels, solutions, suspensions, sticks, sprays, pastes, plasters, and other kinds of transdermal drug delivery systems. The pharmaceutically acceptable carriers or excipients may include emulsifying agents, antioxidants, buffering agents, preservatives, humectants, penetration enhancers, chelating agents, gel-forming agents, ointment bases, perfumes, and skin protective agents.

The pharmaceutical compositions described above for topical administration on the skin may also be used in connection with topical administration onto or close to the part of the body that is to be treated. The compositions may be adapted for direct application or for introduction into relevant orifice(s) of the body (e.g., rectal, urethral, vaginal or oral orifices). The composition may be applied by means of special drug delivery devices such as dressings or alternatively plasters, pads, sponges, strips, or other forms of suitable flexible material.

Controlled Release Percutaneous and Topical Compositions. There are several approaches for providing rate control over the release and transdermal permeation of a drug, including: membrane-moderated systems, adhesive diffusion-controlled systems, matrix dispersion-type systems, and microreservoir systems. A controlled release percutaneous and/or topical composition may be obtained by using a suitable mixture of the above-mentioned approaches.

In a membrane-moderated system, the active drug is present in a reservoir which is totally encapsulated in a shallow compartment molded from a drug-impermeable laminate, such as a metallic plastic laminate, and a rate-controlling polymeric membrane such as a microporous or a non-porous polymeric membrane (e.g., ethylene-vinyl acetate copolymer). The active compound is only released through the rate-controlling polymeric membrane. In the drug reservoir, the active drug substance may either be dispersed in a solid polymer matrix or suspended in a viscous liquid medium such as silicone fluid. On the external surface of the polymeric membrane, a thin layer of an adhesive polymer is applied to achieve an intimate contact of the transdermal system with the skin surface. The adhesive polymer is preferably a hypoallergenic polymer that is compatible with the active drug.

In an adhesive diffusion-controlled system, a reservoir of the active drug is formed by directly dispersing the active drug in an adhesive polymer and then spreading the adhesive containing the active drug onto a flat sheet of substantially drug-impermeable metallic plastic backing to form a thin drug reservoir layer. A matrix dispersion-type system is characterized in that a reservoir of the active drug substance is formed by substantially homogeneously dispersing the active drug substance in a hydrophilic or lipophilic polymer matrix and then molding the drug-containing polymer into a disc with a substantially well-defined surface area and thickness. The adhesive polymer is spread along the circumference to form a strip of adhesive around the disc.

In a microreservoir system, the reservoir of the active substance is formed by first suspending the drug solids in an aqueous solution of water-soluble polymer, and then dispersing the drug suspension in a lipophilic polymer to form a plurality of microscopic spheres of drug reservoirs.

Dosages. The dosage of each compound of the claimed combinations depends on several factors, including: the administration method, the condition to be treated, the severity of the condition, whether the condition is to be treated or prevented, and the age, weight, and health of the person to be treated. Additionally, pharmacogenomic (the effect of genotype on the pharmacokinetic, pharmacodynamic or efficacy profile of a therapeutic) information about a particular patient may affect the dosage used.

As described above, the compounds or compositions described herein may be administered orally in the form of tablets, capsules, elixirs or syrups, or rectally in the form of suppositories. Parenteral administration of a compound is suitably performed, for example, in the form of saline solutions or with the compound incorporated into liposomes. In cases where the compound in itself is not sufficiently soluble to be dissolved, a solubilizer such as ethanol can be applied.

In addition to the foregoing illustrative dosages and dosing protocols, it is to be understood that an effective amount of any one or a mixture of the compounds described herein can be readily determined by the attending diagnostician or physician by the use of known techniques and/or by observing results obtained under analogous circumstances. In determining the effective amount or dose, a number of factors are considered by the attending diagnostician or physician, including, but not limited to the species of mammal, including human, its size, age, and general health, the specific disease or disorder involved, the degree of or involvement or the severity of the disease or disorder, the response of the individual patient, the particular compound administered, the mode of administration, the bioavailability characteristics of the preparation administered, the dose regimen selected, the use of concomitant medication, and other relevant circumstances.

Suitable routes for parenteral administration include intravenous, intraarterial, intraperitoneal, epidurial, intraurethral, intrasternal, intramuscular and subcutaneous, as well as any other art recognized route of parenteral administration. Suitable means of parenteral administration include needle (including microneedle) injectors, needle-free injectors and infusion techniques, as well as any other means of parenteral administration recognized in the art. Parenteral formulations are typically aqueous solutions which may contain excipients such as salts, carbohydrates and buffering agents (preferably at a pH in the range from about 3 to about 9), but, for some applications, they may be more suitably formulated as a sterile nonaqueous solution or as a dried form to be used in conjunction with a suitable vehicle such as sterile, pyrogen-free water. The preparation of parenteral formulations under sterile conditions, for example, by lyophilization, may readily be accomplished using standard pharmaceutical techniques well known to those skilled in the art.

The effective use of the methods described herein for treating or ameliorating MS using one or more compounds described herein may be based upon animal models, such as murine and rabbit models. For example, it is understood that MS in humans are characterized by a loss of function, and/or the development of symptoms, each of which may be elicited in animals, such as mice and rabbits, and other surrogate test animals. Illustrative animal models of Multiple Sclerosis that may be used to evaluate the methods of treatment and the pharmaceutical compositions described herein to determine the therapeutically effective amounts described herein, include but not limited to the mouse SJL, NOD, and C57BL/6J mice (Taconic or The Jackson Laboratory), in which EAE is induced by subcutaneous immunization of 10-week-old NOD mice or 7-week-old C57BL/6J into the flanks with 200 µl of an emulsion containing 150 µg of MOG35-55 peptide (MEVGWYRSPFSRVVHLYRNGK; David Teplow, David Geffen School of Medicine, UCLA, Los Angeles, California, USA) and 400 µg of Mycobacterium tuberculosis extract H37 Ra (Difco) in incomplete Freund's adjuvant oil. In addition, the animals receive 150 ng of pertussis toxin (List Biological Laboratories) i.p. on day 0 and day 2. EAE in SJL mice is induced by immunization with 50 µg of PLP131-151 peptide in complete Freund's adjuvant oil.

The following examples further illustrate specific embodiments of the invention; however, the following illustrative examples should not be interpreted in any way to are to limit invention.

### EXAMPLES

EXAMPLE. The following examples, and corresponding pharmaceutically acceptable salts thereof are described herein, and may be included in the methods and compositions described herein.

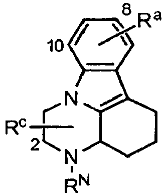

| R^{a} | R^{c} | R^{N} |
|---|---|---|
| 8-Me | H | H |
| 8-OMe | H | Me |
| 8-cyclohexyl | H | H |
| 8-Me | H | Me₂(CH₂)₄NHCH₂CH(OH)CH₂ |
| 8-cyclohexyl | H | 3,4,5-trimethoxybenzoyl |
| 8-Br | H | n-Bu |
| 8-Me | H | CF₃(CF₂)₅C(O) |
| H | H | NH₂CH₂CH₂ |
| 8-(4-methoxyphenyl) | H | H |
| 8-Me | H | 3-methyl-1-piperidinyl-C(O)CH₂ |
| 8-cyclododecyl | H | H |
| 8-Me | H | Me |
| 8-cyclohexyl | H | PhCH=CHCH=NNHC(O)CH₂ |
| 8-Me | H | 4-methyl-1-piperazinyt-CH₂CH(OH)CH₂ |
| 8-Me | H | 4-(3,4-dimethoxyphenyl)thiazol-2-yl |
| 8-F | H | H |
| 8-cyclohexyl | H | 1-methyl-3-nitro-1H-1,2,4-triazol-5-yl |
| 8-(adamant-1-yl) | H | H |
| 8-Me | H | piperidin-1-yl-CH(OH)CH₂ |
| 8-CO₂Et | H | H |
| H | 2-Ph | H |
| 8-cyclohexyl | H | Et₂NCH₂C(O) |
| 6,8-dimethyl | H | H |
| 8-Me | H | 4-ethylpiperazin-1-yl-CH₂CH(OH)CH₂ |
| 8-Me | H | NCCH₂ |
| 8-Me | H | NH₂CH₂CH₂ |
| 8-cyclohexyl | H | Et₂NCH₂CH₂ |
| 8-Me | H | NH₂C(O)CH₂CH₂ |
| 8-Me | H | CH₃C(O)CH₂ |
| 8-Me | H | 2-fluorobenzoyl |
| 8-OAc | H | H |
| 8-Me | H | (i-Pr)₂NCH₂CH₂ |
| 8-C(O)NHNH₂ | H | H |
| 8-Me | H | 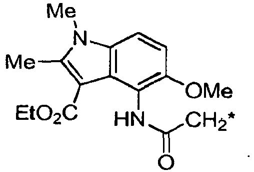 |
| 8-Me | H | t-BuOC(O)NHCH(i-Bu)C(O) |
| 8-Me | H | (S)-t-BuOC(O)NHCH(i-Bu)C(O) |
| 8-Me | H | Me₂N(CH₂)₄NHCH₂CH(OH)CH₂ |
| 8-Me | H | Et₂N(CH₂)₃NHCH₂CH(OH)CH₂ |

EXAMPLE. The following examples, and corresponding pharmaceutically acceptable salts thereof are described herein, and may be included in the methods and compositions described herein.

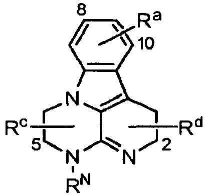

| R^{a} | R^{c} | R^{d} | R^{N} |
|---|---|---|---|
| 9-EtO | H | H | Me |
| 9-MeO | H | H | H |
| 9-MeO | H | H | n-propyl |
| 9-MeO | H | H | Me |
| 9-MeO | H | H | benzyl |
| H | H | H | Me |
| 9-F | H | H | Me |
| 9-MeO | H | H | cyclohexyl, |
| 9-cyclohexyl | H | H | Me |
| 9-Me | H | H | Me |
| 9-hydroxy | H | H | Me |
| 7-MeO | H | H | Me |
| H | H | H | Et |
| 9-cyclohexyl | H | H | H |
| 9-n-PrO | H | H | Me |
| 8-MeO | H | H | Me |
| 9-benzyloxy | H | H | Me |
| H | 5-Me | H | Me |

EXAMPLE. The following examples, and corresponding pharmaceutically acceptable salts thereof are described herein, and may be included in the methods and compositions described herein.

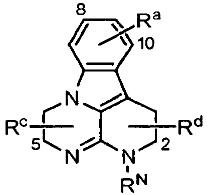

| R^{a} | R^{c} | R^{d} | R^{N} |
|---|---|---|---|
| 9-EtO | H | H | Me |
| 9-MeO | H | H | H |
| 9-MeO | H | H | n-propyl |
| 9-MeO | H | H | Me |
| 9-MeO | H | H | benzyl |
| H | H | H | Me |
| 9-F | H | H | Me |
| 9-MeO | H | H | cyclohexyl |
| 9-cyclohexyl | H | H | Me |
| 9-Me | H | H | Me |
| 9-hydroxy | H | H | Me |
| 7-MeO | H | H | Me |
| H | H | H | Et |
| 9-cyclohexyl | H | H | H |
| 9-n-PrO | H | H | Me |
| 8-MeO | H | H | Me |
| 9-benzyloxy | H | H | Me |
| H | 5-Me | H | Me |

EXAMPLE. The following examples, and corresponding pharmaceutically acceptable salts thereof are described herein, and may be included in the methods and compositions described herein.

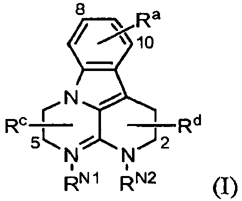

| R^{a} | R^{c} | R^{d} | R^{N1} | R^{N2} |
|---|---|---|---|---|
| 9-EtO | H | H | Me | Me |
| 9-MeO | H | H | H | H |
| 9-MeO | H | H | n-propyl | n-propyl |
| 9-MeO | H | H | Me | Me |
| 9-MeO | H | H | benzyl | benzyl |
| 9-MeO | H | H | Me | benzyl |
| H | H | H | Me | Me |
| 9-F | H | H | Me | Me |
| 9-MeO | H | H | cyclohexyl | cyclohexyl |
| H | 5-Me | H | Me | Me |
| 9-cyclohexyl | H | H | Me | Me |
| 9-Me | H | H | Me | Me |
| 9-hydroxy | H | H | Me | Me |
| 7-MeO | H | H | Me | Me |
| H | H | H | Et | Et |
| 9-cyclohexyl | H | H | H | H |
| 9-n-PrO | H | H | Me | Me |
| 8-MeO | H | H | Me | Me |
| 9-benzyloxy | H | H | Me | Me |

EXAMPLE. Pirlindole, tetrindole, metralindole, or a combination thereof (50 mg) is administered two times daily to a patient suffering from or in need of relief from multiple sclerosis Illustratively, the pirlindole in the form of tablets (comprising 25 mg of pirlindol, 30 mg of lactose, and 5 mg of magnesium stearate) for oral administration. The duration of treatment in this and other examples described herein is determined according to the progression of multiple sclerosis in each individual patient and dose adjustments are made accordingly. Treatment efficacy in this and other examples described herein is monitored by self-reporting and the results of treatment are evaluated statistically using Student's t-test and/or Fisher's "Fi" criterion.

EXAMPLE. Pirlindole, tetrindole, metralindole, or a combination thereof (100 mg) is administered two times daily to an adult or a child.

EXAMPLE. Pirlindole, tetrindole, metralindole, or a combination thereof (200 mg) is administered two times daily to an adult or a child.

EXAMPLE. Pirlindole, tetrindole, metralindole, or a combination thereof (250 mg) is administered two times daily to an adult.

EXAMPLE. Pirlindole, tetrindole, metralindole, or a combination thereof (400 mg) is administered two times daily to an adult.

EXAMPLE. Pirlindole, tetrindole, metralindole, or a combination thereof (25 mg) is administered three times daily to an adult or a child.

EXAMPLE. Pirlindole, tetrindole, metralindole, or a combination thereof (50 mg) is administered three times daily to an adult or a child.

EXAMPLE. Pirlindole, tetrindole, metralindole, or a combination thereof (100 mg) is administered three times daily to an adult or a child.

EXAMPLE. Pirlindole, tetrindole, metralindole, or a combination thereof (200 mg) is administered three times daily to an adult or a child.

EXAMPLE. Pirlindole, tetrindole, metralindole, or a combination thereof (250 mg) is administered three times daily to an adult.

EXAMPLE. Pirlindole, tetrindole, metralindole, or a combination thereof is administered as described herein, such as oral administration of 50 mg, two times daily, and co-administered with oral simvastatin (20 mg tablet, Merck & Co Inc) two times daily.

EXAMPLE. Pirlindole, tetrindole, metralindole, or a combination thereof is administered as described herein, such as oral administration of 50 mg, two times daily, and co-administered with oral glatiramer acetate 20 mg subcutaneously (Teva) one time daily.

EXAMPLE. Pirlindole, tetrindole, metralindole, or a combination thereof is administered as described herein, such as oral administration of 50 mg, two times daily, to a patient diagnosed with Progressive Multiple Sclerosis, and co-administered with oral simvastatin (20 mg tablet, Merck & Co Inc) two times daily, and co-administered with oral glatiramer acetate (20 mg sc inj, Teva) one time daily.

EXAMPLE. Pirlindole, tetrindole, metralindole, or a combination thereof is administered as described herein, such as oral administration of 100 mg, two times daily, to a patient diagnosed with secondary progressive multiple sclerosis, and co-administered with oral memantine. Treatment is started with 50 mg daily of pirlindole (one 25mg tablet in the morning and one in the afternoon or evening) during the 1st week. In the 2nd week 100 mg per day (two tablets in the morning and two in the afternoon or evening) and in the 3rd week 150 mg per day (two tablets in the morning and four tablets in the afternoon or evening) is recommended. From the 4th week on, treatment can be continued with the recommended maintenance dose of 200 mg per day (four tablets twice a day).

EXAMPLE. Pirlindole, tetrindole, metralindole, or a combination thereof is administered as described herein, such as oral administration of 25 mg, two times daily, to a patient diagnosed with secondary progressive multiple sclerosis, and co-administered with oral azathioprine (50 mg tablet, GlaxoSmithKline) two times daily.

EXAMPLE. Pirlindole, tetrindole, metralindole, or a combination thereof is administered as described herein, such as oral administration of 25 mg, two times daily, to a patient diagnosed with relapsing-remitting multiple sclerosis, and co-administered with oral mucophenolate-mofetil (500 mg tablet, Roche) two times daily.

EXAMPLE. Pirlindole, tetrindole, metralindole, or a combination thereof is administered as described herein, such as oral administration of 25 mg, two times daily, to a patient diagnosed with progressive-relapsing multiple sclerosis, and co-administered with oral mucophenolate-mofetil (500 mg tablet, Roche) two times daily.

EXAMPLE. Tetracyclic pyrazinoindoles, and/or pharmaceutically acceptable salts thereof, including the compounds described herein, are efficacious in the mouse MOG-induced model of chronic progressive EAE. Briefly, chronic progressive EAE in SJL mice is induced by immunization with 50 µg of PLP131-151 peptide or with MOG35-55 in complete Freund's adjuvant. Animals are kept in a conventional pathogen-free facility. Pirlindole is given daily beginning on day 20 at 10 mg/kg intravenously. Vehicle consisting of 2% DMSO. Clinical signs of EAE are assessed according to the following score: 0, no signs of disease; 1, loss of tone in the tail; 2, hindlimb paresis; 3, hindlimb paralysis; 4, tetraplegia; 5, moribund. (Forte et al., Cyclophilin D inactivation protects axons in experimental autoimmune encephalomyelitis, an animal model of multiple sclerosis. Proc Natl Acad Sci U S A. 104(18):7558-63 (2007 May 1)).

EXAMPLE. Severe EAE Animal Model of MS. Experimental Autoimmune Encephalomyelitis (EAE), also called Experimental Allergic Encephalomyelitis, can be induced in healthy animals, such as mice, rats, guinea pigs, rabbits, macaques, rhesus monkeys and marmosets, by injecting whole or parts of various proteins that make up myelin, such as Myelin Basic Protein (MBP), Proteolipid Protein (PLP), and Myelin Oligodendrocyte Glycoprotein (MOG). Briefly, to generate severe EAE, 12-week-old C57BL/6 female mice (Jackson Laboratory, Bar Harbor, Maine, USA) are injected subcutaneously at the back of the tail and 7 days later in the flanks with 300 µg of MOG35-55 peptide (Bernard et al., Myelin oligodendrocyte glycoprotein: a novel candidate autoantigen in multiple sclerosis. J Mol Med 75: 77-88 (1997)) emulsified in 100 µl of complete Freund's adjuvant (CFA) (Difco Laboratories, Detroit, Mich., USA) containing an additional 4 mg/ml of Mycobacterium tuberculosis (H37Ra). Mice are injected intraperitoneally (IP) with 300 ng of reconstituted lyophilized pertussis toxin (List Biological Laboratories, Campbell, Calif., USA) in 200 µl of PBS. The pertussis toxin injection is repeated after 48 h (Liu et al. TNF is a potent anti-inflammatory cytokine in autoimmune-mediated demyelination. Nat Med 4: 78-83 (1998)).

EXAMPLE. Evaluation of Tetracyclic Pyrazinoindoles, and Pharmaceutically Acceptable Salts Thereof. Animals are treated IP daily, beginning on the day of MOG induction, with one or more tetracyclic pyrazinoindoles or vehicle (saline). Seven to nine animals per group are used. Control EAE mice are injected with 200 µl saline daily, while the treated EAE mice receive one or more tetracyclic pyrazinoindoles, in the range from about 1 mg/kg to about 50 mg/kg twice a day, from 1 mg/kg to about 25 mg/kg twice a day, or about 1 mg/kg twice a day until they are sacrificed. In a last group of mice, treatment with one or more intraperitoneal tetracyclic pyrazinoindoles (in the range from about 5 mg/kg to about 50 mg/kg daily, from 5 mg/kg to about 25 mg/kg daily, or about 5 mg/kg daily) is initiated only from Day 10 post-immunization, when clinical disease is beginning to be apparent in mice.

All mice are weighed on a daily basis. Severity of EAE is graded according to Liu J, Marino MW, Wong G, Grail D, Dunn A, Bettadapura J, et al. TNF is a potent anti-inflammatory cytokine in autoimmune-mediated demyelination. Nat Med 1998; 4: 78-83). Grades are: 0, no disease; 1, limp tail; 2, partial paralysis of one or two hind limbs; 3, complete paralysis of hind limbs; 4, hind limb paralysis and fore limb paraparesis; 5, moribund.

EXAMPLE. Mild EAE Animal Model of MS. Briefly, to obtain mild EAE, only one injection of MOG35-55 peptide emulsified in 100 µl of CFA is given to 12-week-old C57BL/6 female mice (Jackson Laboratory, Bar Harbor, Maine, USA). Subsequently, the dose is reduced from 300 to 25 µg, and M. tuberculosis is not supplemented to the CFA. Pertussis toxin is continued to be administered as with the severe EAE Animal Model of MS.

EXAMPLE. Acute EAE Murine Model. Experimental Allergic Encephalomyelitis (EAE) is a central nervous system (CNS) autoimmune demyelinating disease that mimics many of the clinical and pathologic features of Multiple Sclerosis (MS). The MOG murine model consists of a sensitization period, induced by the single subcutaneous (SC) injection of MOG emulsified in Complete Freund's Adjuvant (CFA) on study days 0 and 6, followed by IP supplemental immunostimulation with Pertussis Toxin (PT) carried out once at the time of EAE induction and once again 48 hours later. Additional details are described in Gilgun-Sherki et al., Neurosciences Research 47:201-207 (2003).

Briefly, the study is performed in female C57BL/6J mice (Harlan Laboratories Israel, Ltd.) as young adults, 8-9 weeks of age at study initiation. animals are acclimatized to laboratory conditions at least 5 days. During acclimation and throughout the entire study duration, animals are housed in groups of 10 mice maximum in polypropylene cages, which are fitted with solid bottoms and filled with wood shavings as bedding material. Animals are provided ad libitum a commercial rodent diet and free access to drinking water, supplied to each cage via polyethylene bottles with stainless steel sipper tubes. Water is monitored periodically Automatically controlled environmental conditions are set to maintain temperature at 20-24°C with a relative humidity (RH) of 30-70%, a 12:12 hour light: dark cycle and 15-30 air changes/hr in the study room. Temperature and RH are monitored daily. The light cycle is monitored by the control clock. Weight variation of animals at the time of treatment initiation should not exceed ± 20% of the mean weight. At the end of the study, surviving animals are euthanized by CO₂ asphyxiation.

Antigen. The MOG peptide (35-55 human, [H]-MEVGWYRPFSRVVHLYRNGK-[OH]) solution is freshly prepared prior to each inoculation session by dissolving the RP-HPLC-purified lyophilized powder in Phosphate Buffered Saline (PBS) to achieve a solution at a final injected concentration of 2 mg/ml. This concentration is appropriate for the selected dose and dose volume of 200 µg MOG in 100 µL PBS.

Sensitizer. Complete Freund's Adjuvant (CFA) suspension in mineral oil containing heat killed Mycobacterium Tuberculosis H37 Ra at a concentration of 3 mg/mL is used as supplied.

MOG/CFA Emulsion. Prior to each inoculation carried out on study days 0 and 6, 100 µl of MOG solution (200µg) is emulsified with 100µl of CFA suspension (300µg heat killed Mycobacterium Tuberculosis H37 Ra). The solution is thoroughly mixed by employing two syringes connected by a Luer fitting to equal a total dose volume of 200 µl/animal.

Immunostimulant. Prior to the first immunostimulation injection performed on study day 0, the Pertussis Toxin (PT) stock solution (Clear liquid exotoxin, produced by Bordetella Pertussis, 0.2 mg/ml, Sigma-Aldrich) is freshly prepared by diluting the commercial PT sample (0.2 mg/mL, total of 50 µg /250 µL / vial) in sterile distilled water (Water for Injection) to achieve a stock concentration of 100 µg/mL.

Prior to each injection on study days 0 and 2, the PT stock solution (100 µg/mL) is diluted in Phosphate Buffered Saline (PBS) to achieve a final injected concentration of 2 µg/ml, which is appropriate for the selected dose level and volume dosage.

Following each PT injection, the daily vial is discarded. Diluted stock solution (100 µg/mL) is kept refrigerated (2-8 °C) and is reused for the second PT injection on study day 2. The solution is not used if more than 48 hours have passed following its preparation. Thorough vortexing is required just prior to each PT injection session.

All animals are subjected to a single inoculum injection of MOG/CFA on study days 0 (study commencement) and 6. The inoculum injection consists of a homogenate emulsive mixture of MOG and CFA as follows, MOG/CFA encephalitogenic emulsive inoculum (200µg MOG / 300µg CFA) is injected at a total dose volume of 200 µL/animal and is delivered as 2× 100µL subcutaneous (SC) bilateral injections over the paralumbar regions.

In order to increase the permeability of the blood-brain barrier (BBB), all animals are subjected to supplemental immunostimulation by Pertussis Toxin (PT), which is administered at a dose level of 20 µg/kg (approximately 400 ng/mouse), by intraperitoneal (IP) injections at 2 occasions: once at the time of EAE induction on study day 0 and once 48 hours later (study day 2). Pertussis Toxin solution is injected at a volume dosage of 10 mL/kg.

Initially, all animals are examined for signs of any neurological responses and symptoms prior to EAE induction (study day 0) and are examined on a daily basis throughout the 35-day observation period. EAE reactions are scored and recorded according to a 0-15 scale Clinical signs Scoring Methods.

| | Signs/Symptoms | Grade |
|---|---|---|
| Tail | No signs | 0 |
| | Half paralyzed tail | 1 |
| | Fully paralyzed tail | 2 |
| For each hind or forelimb | No signs | 0 |
| | Weak or altered gait | 1 |
| | Paresis | 2 |
| | Fully paralyzed limb | 3 |
| | Mortality | 15 |

The clinical Score is determined by summing the score of each section (Weaver at al, 2005).

Throughout the 35-day study, clinical examinations are performed and recorded at least once daily in addition to the EAE clinical scoring and assessment. Observations include changes in skin, fur, eyes, mucous membranes, occurrence of secretions and excretions (e.g. diarrhea), autonomic activity (e.g. lacrimation, salivation, piloerection, pupil size, unusual respiratory pattern), gait, posture, and response to handling, as well as the presence of unusual behavior, tremors, convulsions, sleep, and coma.

EXAMPLE. Pirlindole. The test compounds are made as a weekly concentrated stock solution, aliquoted in 7 vials and frozen at -20 °C. One vial is thawed for daily administration. Aliquot preparation is performed once every week. Dexamethasone (positive control, Sigma), is diluted in Ethanol to achieve a concentration of 1 mg/mL and diluted with distilled water to achieve the dose concentration of 0.05 mg/mL. Vehicle, test compounds, and dexamethasone positive control are administered once daily beginning on study day 0.

**Table. Treatment Groups**

| Group | Group size (n) | Test Material | Route | Dose Level (mg/kg/admin) | Volume dosage (mL/kg) |
|---|---|---|---|---|---|
| A | 10 | Vehicle Control | IP | 0 | 10 |
| B | 10 | Positive Control (Dexamethasone) | IP | 1.0 | 10 |
| C | 10 | Pirlindole | IP | 1.0 | 10 |
| D | 10 | Pirlindole | IP | 3.0 | 10 |
| E | 10 | Pirlindole | IP | 10 | 10 |
| F | 10 | Pirlindole | IP | 30 | 10 |

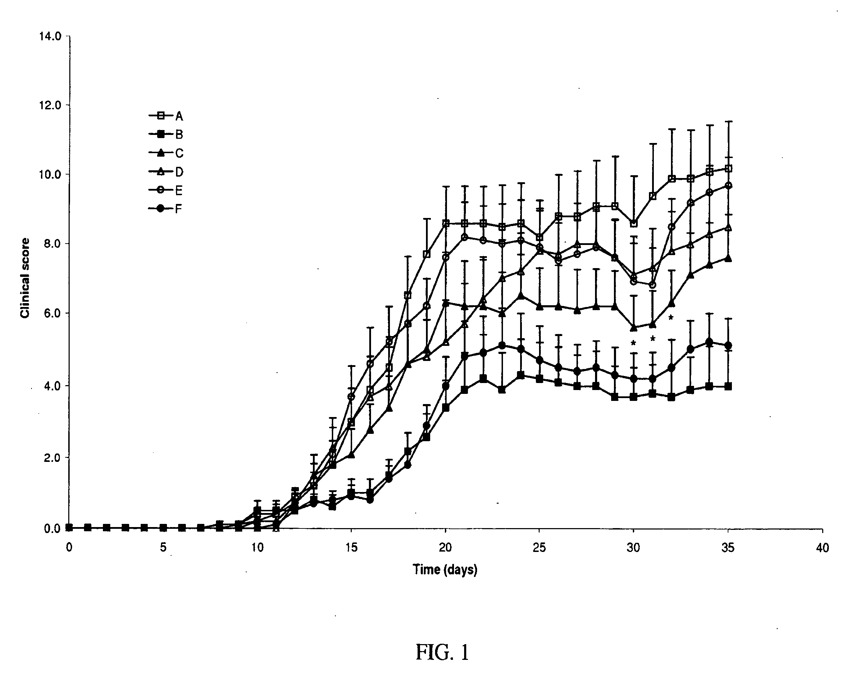

The results are shown in FIG. 1. The activity observed for the positive control (B) was statistically significant compared to the vehicle-only treatment group (A), p<0.01, starting at day 15 and continuing throughout the study. The activity observed for the treatment group (E) was statistically significant compared to the vehicle-only treatment group (A), p<0.01, starting at day 15 and continuing throughout the study (with the exception of day 23, p<0.05). Though the activity observed for the treatment group (C) was not statistically significant compared to the vehicle-only treatment group (A) at the end of the study, the intermediate time points on days 30, 31, and 32 were statistically significant compared to the vehicle-only treatment group (A), p<0.05.

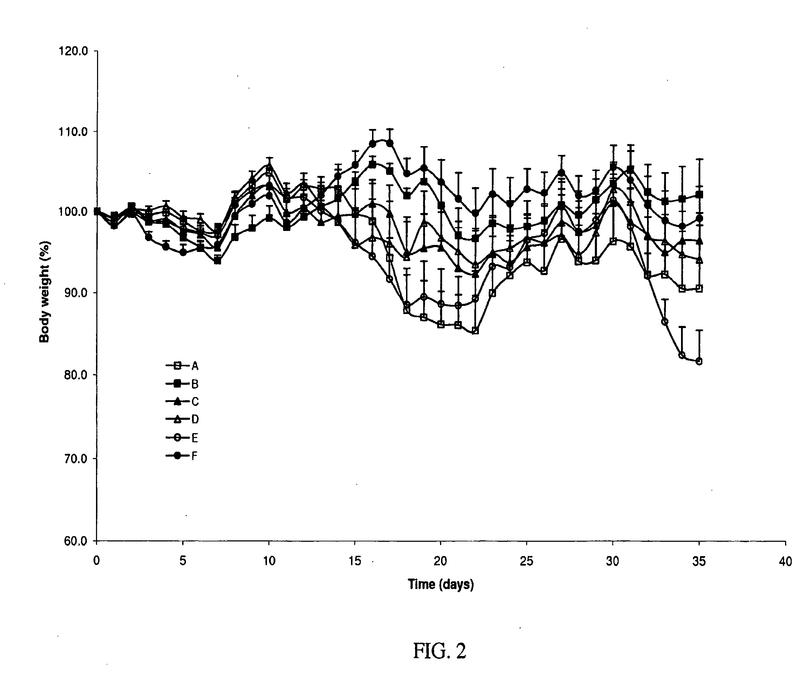

Body weight loss can be the first sign of disease initiation, while a sudden marked weight gain tends to accompany remission of EAE symptoms. Therefore, determination of individual body weights of animals is made shortly before EAE induction on study day 0 (study commencement) and is monitored on a daily basis throughout the 35-day observation period. The results are shown in FIG. 2.

EXAMPLE. Histological Analysis. Anaesthetized mice are perfused with 40 ml of cold saline and the CNS is dissected. The sacral part of the spinal cord is immersed in 4% paraformaldehyde overnight and embedded in paraffin wax, cross sectioned at 6-8 µm and stained with Luxolfast blue for evidence of demyelination, and optionally stained with hematoxylin and eosin (H&E) for evidence of inflammation. Optic nerves are fixed in 2.5% glutaraldehyde, embedded in epon, sectioned at 2 µm, and stained with toluidine blue.

The vehicle-treated EAE mice, and a first group of mice treated with one or more compounds described herein are sacrificed at Days 19-21 after MOG immunization. A second group of treated mice are killed at Day 27 when disease is full-blown. The anterior (Ant), lateral (Lat) and posterior (Post) columns of the right and left cord are evaluated blind, using a scale from 0 (no inflammation) to 3 (severe inflammation deep in the CNS parenchyma and which approaches grey matter areas).

EXAMPLE. Histological Analysis of mice treated with pirlindole (BVA-201). Briefly, all samples are longitudinal sections of the vertebral column and spinal cord stained with hematoxylin and eosin (H&E). Sample A is the cranial part of the vertebral column and sample B is the caudal part. The modifiers F-focal, MF-multifocal, and D-diffuse are used to describe the distribution of inflammatory cellular infiltration. Diffuse is used when the lesion is continuous (at least on one side) and affects the entire SC. In some cases the lesion affects the entire SC (100%) but its distribution is multifocal because it is not contiguous i.e. there are small unaffected segments on one side, which are affected on the contralateral side.

Histology analysis refers to 2 parameters: (a) Severity Score which is referring mainly but not limited to infiltration on inflammatory cells; and (b) White Matter Damage (WMD). Codes: Dist - distribution; GM - gray matter; IC - inflammatory cells; ND - not done; SC - spinal cord; Sev - severity; WM - white matter; WMD - white matter damage; WNL - within normal limits. Severity scores 0 - WNL, 1 - minimal, 2- mild, 3- moderate, 4-severe. Half grades are also used. Severity refers to the amount of inflammatory infiltration and WMD. If there is a discrepancy in the grade of these 2 changes, then this is noted.

The extent of WMD is indicated in percentage referring to the amount of affected spinal cord tissue in the sample. In many cases the damage affected one side (dorsal or ventral) of the SC only. In some cases, when the plane of section is more lateral the WMD affects both sides. The available SC sample is considered 100%. Thus, if in a 5cm long SC, there are changes in a 0.5cm long segment, then this was noted as 10% irrespective of whether the damage affected one side or both. Although not reflected in the percentage, the extent of the lesion is taken into consideration in evaluation of the severity. WMD describes a set of changes including vacuolation, gliosis, and presence of myelin debris. In a few cases these changes have the typical morphology of Wallerian degeneration (rows of digestion chambers). In most samples, the nature of the damage could be Wallerian degeneration, demyelination, or both, and/or non-specific damage associated with inflammation.

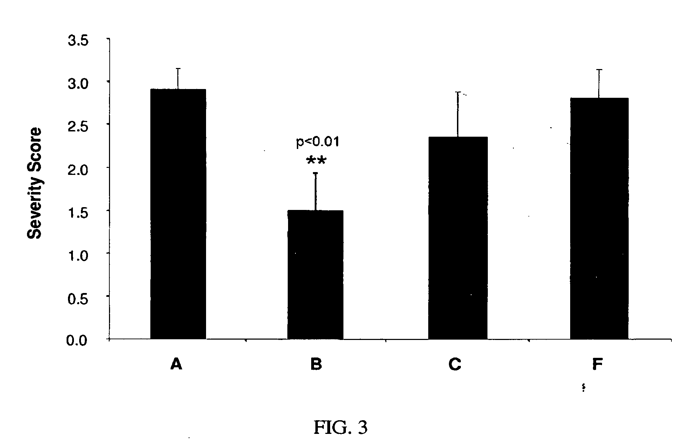

Individual animal data for severity score is averaged and summarized in FIG. 3. Treatment groups: (A)-vehicle control; (B)-dexamethasone; (C)-BVA-201 at 1 mg/kg; (F)-BVA-201 at 30 mg/kg. The only active compound in reducing the severity score was dexamethasone. No difference in severity score was found between the BVA-201 treated groups and vehicle. These data suggest that BVA-201 does not have any anti inflammatory effect.

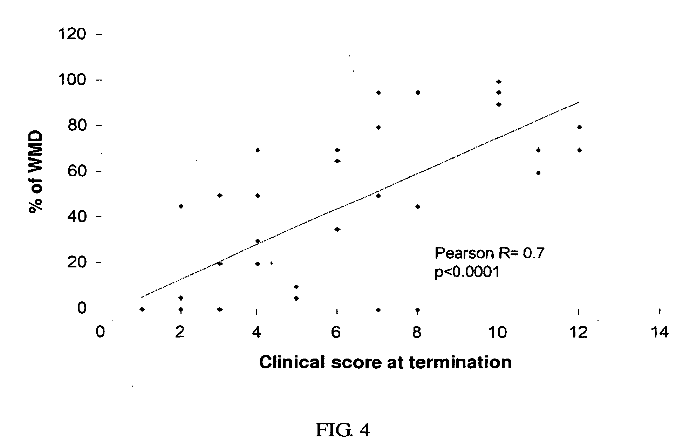

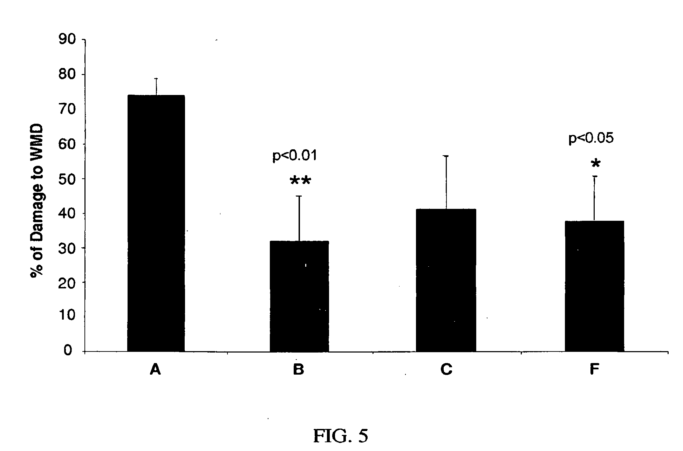

Individual animal data for WMD is averaged and summarized in FIG. 4 and FIG. 5. A positive and highly significant correlation was found between the WMD and the clinical score determined at termination (FIG. 4). A significant protective effect by BVA-201 was found following treatment with 30 mg/kg (FIG 5). As the positive control, a significant protective effect was also found following treatment with dexamethason (FIG. 5). Without being bound by theory, it is believed herein that together, these data suggest that the dexamethasone activity in preventing WMD is mediated by its anti inflammatory effect, whereas the protective efficacy of BVA-201 is not related to anti-inflammatory effect but rather to direct or other indirect protection of the neurons. In the case of direct and/or indirect protection of the neurons, without being bound by theory, it is believed herein that the compounds, compositions, and methods described herein may directly cause myelin repair or remyelination, or alternatively protect axons, and allow other processes to more effectively cause myelin repair or remyelination. For example, the compounds, compositions, and methods described herein may protect oligodendrocytes (myelin producing cells). Without being bound by theory, overall it is believed herein that the data also suggest mitochondrial stabilization by the compounds, compositions, and methods described herein.

In general, the data show one or more of (a) less demyelination, (b) improved myelin repair, and/or (c) remyelination in the BVA-201 treatment group compared to the vehicle-only treatment group.

EXAMPLE. Equivalent models of MS using SJL/J Mouse (Jackson Laboratories) EAE and Dark Agouti (DA) Rat (Harlan) EAE are also described. Details of the models are described in U.S. Patent No. 7,041,661, the disclosure of which is incorporated herein by reference.

It is to be understood that the animal model and other assay Examples described herein for pirlindole may be used to assay and/or evaluate the various co-therapies and co-administrations described herein, including co-administration with one or more NMDA receptor antagonists, HMG-CoA reductase inhibitors, and the like, and combinations thereof.

## Claims

1. A compound for use in treating multiple sclerosis comprising the formula: 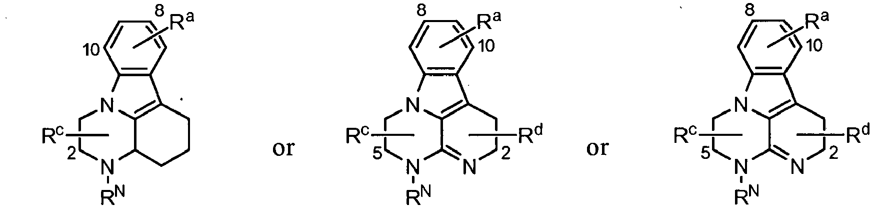 or a pharmaceutically acceptable salt thereof; wherein
R^{a} is hydrogen, or R^{a} represents 1 to 4 substituents, each independently selected from halo and hydroxy, and alkoxy, acyloxy, alkyl, heteroalkyl, cycloalkyl, cycloheteroalkyl, aryl, heteroaryl, arylalkyl, or heteroarylalkyl, each of which is optionally substituted;
R^{c} is hydrogen, or R^{c} represents 1 or 2 substituents, each independently selected from alkyl, heteroalkyl, cycloalkyl, cycloheteroalkyl, aryl, heteroaryl, arylalkyl, and heteroarylalkyl, each of which is optionally substituted;
R^{d} is hydrogen, or R^{d} represents 1 or 2 substituents, each independently selected from alkyl, heteroalkyl, cycloalkyl, cycloheteroalkyl, aryl, heteroaryl, arylalkyl, and heteroarylalkyl, each of which is optionally substituted; and
R^{N} is hydrogen or hydroxy, or alkoxy, acyloxy, alkyl, heteroalkyl, cycloalkyl, cycloheteroalkyl, aryl, heteroaryl, arylalkyl, or heteroarylalkyl, each of which is optionally substituted; or R^{N} and the attached nitrogen form an amide, carbamate, or urea, or thiono derivative thereof.

2. The compound for use according to claim 1 comprising the formula: 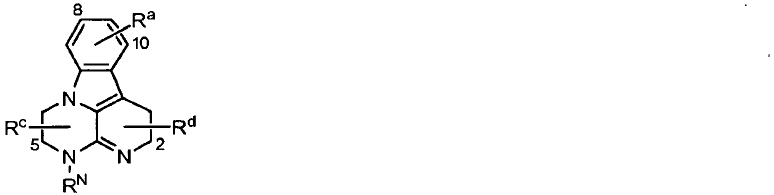 or a pharmaceutically acceptable salt thereof.

3. The compound for use according to claim 1 selected from pirlindole, tetrindole, metralindole and pharmaceutically acceptable salts thereof.

4. The compound for use according to any one of claims 1 to 3 co-administered with one or more compounds of the formula: 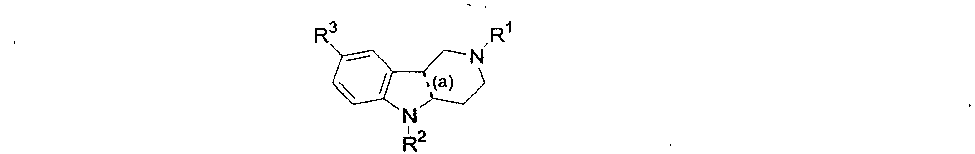 or a pharmaceutically acceptable salt thereof,
wherein R¹ is hydrogen, or alkyl, heteroalkyl, cycloalkyl, cycloheteroalkyl, aryl, heteroaryl, arylalkyl, or heteroarylalkyl, each of which is optionally substituted; or R¹ and the attached nitrogen form an amide, carbamate, or urea, or thiono derivative thereof or arylalkyl;
R² is hydrogen, or alkyl or arylalkyle, each of which is optionally substituted;
R³ is hydrogen, halo, or hydroxy, or alkoxy, acyloxy, alkyl, heteroalkyl, cycloalkyl, cycloheteroalkyl, aryl, heteroaryl, arylalkyl, or heteroarylalkyl, each of which is optionally substituted; and
bond (a) is a single bond or a double bond.

5. The compound for use according to claims 4 selected from dimebon or pharmaceutically acceptable salts thereof.

6. The compound for use according to any one of claims 1 to 3 co-administered with one or more NMDA receptor antagonists.

7. The compound for use according to claim 6 wherein at least one NMDA receptor antagonist is selected from amantadine, dextromethorphan, dextrorphan, ibogaine, ketamine, phencyclidine, riluzole, tiletamine, memantine and remacemide, and pharmaceutically acceptable salts thereof.

8. The compound for use according to claim 7 wherein at least one NMDA receptor antagonist is selected from riluzole, memantine, amantadine, and dextromethorphan, and pharmaceutically acceptable salts thereof.

9. The compound for use according to any one of claims 1 to 3 co-administered with one or more HMG-CoA reductase inhibitors.

10. The compound for use according to claim 9 wherein at least one HMG-CoA reductase inhibitor is selected from simvastatin, pravastatin, lovastatin, fluvastatin, atorvastatin, rosuvastatin, and cerivastatin, and pharmaceutically acceptable salts thereof.

11. The compound for use according to claim 10 wherein at least one HMG-CoA reductase inhibitor is selected from simvastatin and lovastatin, and pharmaceutically acceptable salts thereof.

12. The compound for use according to any one of claims 1 to 3 co-administered with one or more immunosuppressive drugs.

13. The compound for use according to claim 12 wherein at least one immunosuppressive drug is selected from corticosteroids, cyclophophosphamide, methotrexate, azathioprine, mucophenolate mofetil, cyclosporine, mitoxantrone, natalizumab, daclizumab, alemtuzumab, rituximab, and pharmaceutically acceptable salts thereof.

14. The compound for use according to any one of claims 1 to 3 co-administered with one or more immunomodulatory drugs.

15. The compound for use according to claim 14 wherein at least one immunomodulatory drug is selected from interferon beta-lb, interferon beta-la, glatiramer acetate (copaxone), natalizumab, rituximab, daclizumab, BG12, fingolimod, laquinimod, and pharmaceutically acceptable salts thereof.

## Patentansprüche

1. Verbindung zur Verwendung in der Behandlung von Multipler Sklerose umfassend die Formel: 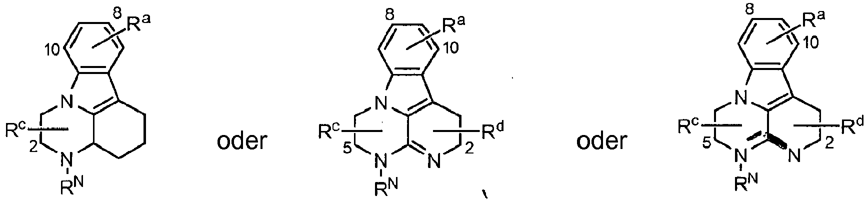 oder ein pharmazeutisch verträgliches Salz davon; wobei
R^{a} Wasserstoff ist, oder R^{a} 1 bis 4 Substituenten darstellt, jeder unabhängig ausgewählt aus Halo und Hydroxy, und Alkoxy, Acyloxy, Alkyl, Heteroalkyl, Cycloalkyl, Cycloheteroalkyl, Aryl, Heteroaryl, Arylalkyl, oder Heteroarylalkyl, von denen jedes wahlweise substituiert ist;
R^{c} Wasserstoff ist, oder R^{c} 1 oder 2 Substituenten darstellt, jeder unabhängig ausgewählt aus Alkyl, Heteroalkyl, Cycloalkyl, Cycloheteroalkyl, Aryl, Heteroaryl, Arylalkyl, und Heteroarylalkyl, von denen jedes wahlweise substituiert ist;
R^{d} Wasserstoff ist, oder R^{d} 1 oder 2 Substituenten darstellt, jeder unabhängig ausgewählt aus Alkyl, Heteroalkyl, Cycloalkyl, Cycloheteroalkyl, Aryl, Heteroaryl, Arylalkyl, und Heteroarylalkyl, von denen jedes wahlweise substituiert ist; und
R^{N} Wasserstoff oder Hydroxy, oder Alkoxy, Acyloxy, Alkyl, Heteroalkyl, Cycloalkyl, Cycloheteroalkyl, Aryl, Heteroaryl, Arylalkyl, oder Heteroarylalkyl ist, von denen jedes wahlweise substituiert ist; oder R^{N} und das damit verbundene Stickstoff ein Amid, Carbamat, oder Harnstoff, oder ein Thionoderivat davon bilden.

2. Verbindung zur Verwendung gemäß Anspruch 1, umfassend die Formel: 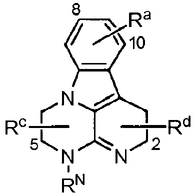 oder ein pharmazeutisch verträgliches Salz davon.

3. Verbindung zur Verwendung gemäß Anspruch 1, ausgewählt aus Pirlindol, Tetrindol, Metralindol und pharmazeutisch verträglichen Salzen davon.

4. Verbindung zur Verwendung gemäß einem der Ansprüche 1 bis 3, zusammen verabreicht mit einer oder mehreren Verbindungen der Formel: 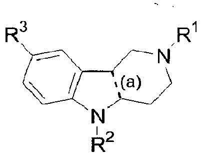 oder einem pharmazeutisch verträglichen Salz davon,
wobei R¹ Wasserstoff, oder Alkyl, Heteroalkyl, Cycloalkyl, Cycloheteroalkyl, Aryl, Heteroaryl, Arylalkyl, oder Heteroarylalkyl ist, von denen jedes wahlweise substituiert ist; oder R¹ und das damit verbundene Stickstoff ein Amid, Carbamat, oder Harnstoff, oder ein Thionoderivat davon, oder Arylalkyl bilden;
R² Wasserstoff, oder Alkyl oder Arylalkyl ist, von denen jedes wahlweise substituiert ist;
R³ Wasserstoff, Halo, oder Hydroxy, oder Alkoxy, Acyloxy, Alkyl, Heteroalkyl, Cycloalkyl, Cycloheteroalkyl, Aryl, Heteroaryl, Arylalkyl, oder Heteroarylalkyl ist, von denen jedes wahlweise substituiert ist; und
Bindung (a) eine Einfachbindung oder eine Doppelbindung ist.

5. Verbindung zur Verwendung gemäß Anspruch 4, ausgewählt aus Dimebon oder pharmazeutisch verträglichen Salzen davon.

6. Verbindung zur Verwendung gemäß einem der Ansprüche 1 bis 3, zusammen verabreicht mit einem oder mehreren NMDA-Rezeptor-Antagonisten.

7. Verbindung zur Verwendung gemäß Anspruch 6, wobei mindestens ein NMDA-Rezeptor-Antagonist aus Amantadin, Dextromethorphan, Dextrorphan, Ibogain, Ketamin, Phencyclidin, Riluzol, Tiletamin, Memantin, und Remacemid, und pharmazeutisch verträglichen Salzen davon ausgewählt ist.

8. Verbindung zur Verwendung gemäß Anspruch 7, wobei mindestens ein NMDA-Rezeptor-Antagonist aus Riluzol, Memantin, Amantadin, und Dextromethorphan, und pharmazeutisch verträglichen Salzen davon ausgewählt ist.

9. Verbindung zur Verwendung gemäß einem der Ansprüche 1 bis 3, zusammen verabreicht mit einem oder mehreren HMG-CoA-Reduktase-lnhibitoren.

10. Verbindung zur Verwendung gemäß Anspruch 9, wobei mindestens ein HMG-CoA-Reduktase-Inhibitor aus Simvastatin, Pravastatin, Lovastatin, Fluvastatin, Atorvastatin, Rosuvastatin, und Cerivastatin, und pharmazeutisch verträglichen Salzen davon ausgewählt ist.

11. Verbindung zur Verwendung gemäß Anspruch 10, wobei mindestens ein HMG-CoA-Reduktase-Inhibitor aus Simvastatin und Lovastatin, und pharmazeutisch verträglichen Salzen davon ausgewählt ist.

12. Verbindung zur Verwendung gemäß einem der Ansprüche 1 bis 3, zusammen verabreicht mit einem oder mehreren immunsuppressiven Arzneimitteln.

13. Verbindung zur Verwendung gemäß Anspruch 12, wobei mindestens ein immunsuppressives Arzneimittel aus Corticosteroiden, Cyclophosphamid, Methotrexat, Azathioprin, Mucophenolatmofetil, Cyclosporin, Mitoxantron, Natalizumab, Daclizumab, Alemtuzumab, Rituximab, und pharmazeutisch verträglichen Salzen davon ausgewählt ist.

14. Verbindung zur Verwendung gemäß einem der Ansprüche 1 bis 3, zusammen verabreicht mit einem oder mehreren immunmodulatorischen Arzneimitteln.

15. Verbindung zur Verwendung gemäß Anspruch 14, wobei mindestens ein immunmodulatorisches Arzneimittel aus Interferon beta-1b, Interferon beta-1a, Glatirameracetat (Copaxon), Natalizumab, Rituximab, Daclizumab, BG12, Fingolimod, Laquinimod, und pharmazeutisch verträglichen Salzen davon ausgewählt ist.

## Revendications

1. Composé destiné à une utilisation dans le traitement de la sclérose en plaques comprenant la formule : 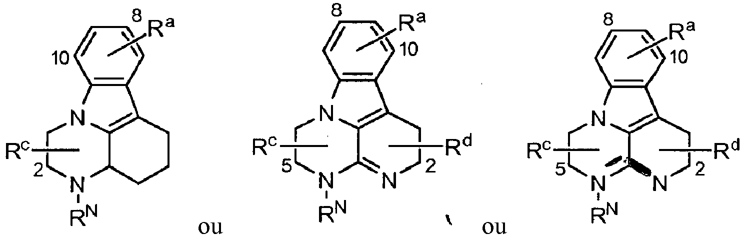 ou un sel pharmaceutiquement acceptable de celui-ci ; dans lequel
R^{a} est un atome d'hydrogène ou R^{a} représente 1 à 4 substituants, chacun indépendamment choisi parmi halogéno et hydroxy, et alcoxy, acyloxy, alkyle, hétéroalkyle, cycloalkyle, cyclohétéroalkyle, aryle, hétéroaryle, arylalkyle ou hétéroarylalkyle, chacun étant éventuellement substitué ;
R^{c} est un atome d'hydrogène ou R^{c} représente 1 ou 2 substituants, chacun indépendamment choisi parmi alkyle, hétéroalkyle, cycloalkyle, cyclohétéroalkyle, aryle, hétéroaryle, arylalkyle et hétéroarylalkyle, chacun étant éventuellement substitué ;
R^{d} est un atome d'hydrogène ou R^{d} représente 1 ou 2 substituants, chacun indépendamment choisi parmi alkyle, hétéroalkyle, cycloalkyle, cyclohétéroalkyle, aryle, hétéroaryle, arylalkyle et hétéroarylalkyle, chacun étant éventuellement substitué ; et
R^{N} est un atome d'hydrogène ou alcoxy, acyloxy, alkyle, hétéroalkyle, cycloalkyle, cyclohétéroalkyle, aryle, hétéroaryle, arylalkyle ou hétéroarylalkyle, chacun étant éventuellement substitué ; où R^{N} et l'atome d'azote fixé forment un amide, un carbamate ou une urée, ou un dérivé thiono de celui-ci.

2. Composé destiné à une utilisation selon la revendication 1 comprenant la formule : 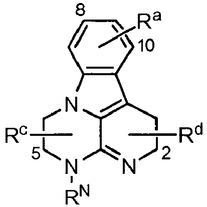 ou un sel pharmaceutiquement acceptable de celui-ci.

3. Composé destiné à une utilisation selon la revendication 1, choisi parmi le pirlindole, le tétrindole, le métralindole et les sels pharmaceutiquement acceptables de ceux-ci.

4. Composé destiné à une utilisation selon l'une quelconque des revendications 1 à 3, administré conjointement à un ou plusieurs composés de formule : 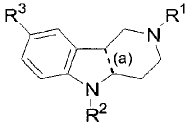 ou un sel pharmaceutiquement acceptable de ceux-ci,
dans laquelle R¹ est un atome d'hydrogène, ou alkyle, hétéroalkyle, cycloalkyle, cyclohétéroalkyle, aryle, hétéroaryle, arylalkyle ou hétéroarylalkyle, chacun étant éventuellement substitué ; où R¹ et l'atome d'azote fixé forment un amide, un carbamate ou une urée, ou un dérivé thiono de celui-ci, ou arylalkyle ;
R² est un atome d'hydrogène ou alkyle ou arylalkyle, chacun étant éventuellement substitué ;
R³ est un atome d'hydrogène, halogéno, hydroxy, ou alcoxy, acyloxy, alkyle, hétéroalkyle, cycloalkyle, cyclohétéroalkyle, aryle, hétéroaryle, arylalkyle ou hétéroarylalkyle, chacun étant éventuellement substitué ; et
la liaison (a) est une liaison simple ou une double liaison.

5. Composé destiné à une utilisation selon la revendication 4 choisi parmi le dimébone ou des sels pharmaceutiquement acceptables de celui-ci.

6. Composé destiné à une utilisation selon l'une quelconque des revendications 1 à 3, administré conjointement à un ou plusieurs antagonistes du récepteur de NMDA.

7. Composé destiné à une utilisation selon la revendication 6, dans lequel au moins un antagoniste du récepteur de NMDA est choisi parmi l'amantadine, le dextrométhorphan, le dextrorphan, l'ibogaïne, la kétamine, la phéncyclidine, le riluzole, la tilétamine, la mémantine et le rémacémide, et les sels pharmaceutiquement acceptables de ceux-ci.

8. Composé destiné à une utilisation selon la revendication 7, dans lequel au moins un antagoniste du récepteur de NMDA est choisi parmi le riluzole, la mémantine, l'amantadine et le dextrométhorphan, et les sels pharmaceutiquement acceptables de ceux-ci.

9. Composé destiné à une utilisation selon l'une quelconque des revendications 1 à 3, administré conjointement à un ou plusieurs inhibiteurs de la HMG-CoA réductase.

10. Composé destiné à une utilisation selon la revendication 9, dans lequel au moins un inhibiteur de la HMG-CoA réductase est choisi parmi la simvastatine, la pravastatine, la lovastatine, la fluvastatine, l'atorvastatine, la rosuvastatine et la cérivastatine, et les sels pharmaceutiquement acceptables de ceux-ci.

11. Composé destiné à une utilisation selon la revendication 10, dans lequel au moins un inhibiteur de la HMG-CoA réductase est choisi parmi la simvastatine et la lovastatine, et les sels pharmaceutiquement acceptables de ceux-ci.

12. Composé destiné à une utilisation selon l'une quelconque des revendications 1 à 3, administré conjointement à un ou plusieurs médicaments immunosuppresseurs.

13. Composé destiné à une utilisation selon la revendication 12, dans lequel au moins médicament immunosuppresseur est choisi parmi les corticostéroïdes, le cyclophophosphamide, le méthotrexate, l'azathioprine, le mucophénolate de mofétil, la cyclosporine, la mitoxantrone, le natalizumab, le daclizumab, l'alemtuzumab, le rituximab, et les sels pharmaceutiquement acceptables de ceux-ci.

14. Composé destiné à une utilisation selon l'une quelconque des revendications 1 à 3, administré conjointement à un ou plusieurs médicaments immunomodulateurs.

15. Composé destiné à une utilisation selon la revendication 14, dans lequel au moins médicament immunomodulateur est choisi parmi l'interféron bêta-1b, l'interféron bêta-1a, l'acétate de glatiramère (copaxone), le natalizumab, le rituximab, le daclizumab, BG12, le fingolimod, le laquinimod, et les sels pharmaceutiquement acceptables de ceux-ci.
